# EUROPEAN PATENT SPECIFICATION

(11) **EP 4 116 393 B1**
(45) Date of publication and mention of the grant of the patent: **01.04.2026**
(21) Application number: 21765224.7
(22) Date of filing: 16.02.2021
(51) Int. Cl.: C09K 11/06, H10K 85/60, H10K 50/15

(54) **ORGANIC ELECTROLUMINESCENT ELEMENT**
ORGANISCHES ELEKTROLUMINESZENZELEMENT
ÉLÉMENT ÉLECTROLUMINESCENT ORGANIQUE

(30) Priority: 03.03.2020 JP 2020035513
(43) Date of publication of application: 11.01.2023
(73) Proprietor: Hodogaya Chemical Co., Ltd., Tokyo, 105-0021 (JP); SFC Co., Ltd., Chungcheongbuk-do (KR)
(72) Inventor: IZUMIDA, Junichi, Tokyo 105-0021 (JP); KASE, Kouki, Tokyo 105-0021 (JP); YAMAMOTO, Takeshi, Tokyo 105-0021 (JP); SURUGA, Kazuyuki, Tokyo 105-0021 (JP); HAYASHI, Shuichi, Tokyo 105-0021 (JP); YOKOYAMA, Norimasa, Tokyo 105-0021 (JP); CHA, Soon-wook, Cheongju-si, Chungcheongbuk-do (KR); JOO, Sung-hoon, Cheongju-si, Chungcheongbuk-do (KR); YANG, Byung-sun, Cheongju-si, Chungcheongbuk-do (KR); KIM, Ji-hwan, Cheongju-si, Chungcheongbuk-do (KR)
(74) Representative: Müller-Boré & Partner Patentanwälte PartG mbB
(86) International application number: PCT/JP2021/005693
(87) International publication number: WO 2021/177022

(56) References cited:
- WO-A1-2015/102118
- WO-A1-2019/135665
- WO-A1-2019/164331
- WO-A1-2019/164331
- WO-A1-2020/004235
- WO-A1-2020/004235
- WO-A1-2020/251049
- CN-A- 110 790 782
- JP-A- 2010 053 131
- KR-A- 20190 003 329
- US-A1- 2015 236 274

## Description

### Technical Field

The present invention relates to an organic electroluminescent device which is a preferred self-luminous device for various display devices. Specifically, this invention relates to organic electroluminescent devices (hereinafter referred to as organic EL devices) using specific arylamine compounds.

### Background Art

The organic EL device is a self-luminous device and has been actively studied for their brighter, superior visibility and the ability to display clearer images in comparison with liquid crystal devices.

In 1987, C. W. Tang and colleagues at Eastman Kodak developed a laminated structure device using materials assigned with different roles, realizing practical applications of an organic EL device with organic materials. These researchers laminated an electrontransporting phosphor and a hole-transporting organic substance, and injected both charges into a phosphor layer to cause emission in order to obtain a high luminance of 1,000 cd/m² or more at a voltage of 10 V or less (refer to Patent Documents 1 and 2, for example).

To date, various improvements have been made for practical applications of the organic EL device. Various roles of the laminated structure are further subdivided to provide an EL device that includes an anode, a hole injection layer, a hole transport layer, a light emitting layer, an electron transport layer, an electron injection layer, and a cathode successively formed on a substrate, and high efficiency and durability have been achieved by the EL device (refer to Non-Patent Document 1, for example).

Further, there have been attempts to use triplet excitons for further improvements of luminous efficiency, and the use of a phosphorescence-emitting compound has been examined (refer to Non-Patent Document 2, for example).

Devices that use light emission caused by thermally activated delayed fluorescence (TADF) have also been developed. In 2011, Adachi et al. at Kyushu University, National University Corporation realized 5.3% external quantum efficiency with a device using a thermally activated delayed fluorescent material (refer to Non-Patent Document 3, for example).

The light emitting layer can be also fabricated by doping a charge-transporting compound generally called a host material, with a fluorescent compound, a phosphorescence-emitting compound, or a delayed fluorescent-emitting material. As described in the Non-Patent Document, the selection of organic materials in an organic EL device greatly influences various device characteristics such as efficiency and durability (refer to Non-Patent Documents 1 to 3, for example).

In an organic EL device, charges injected from both electrodes recombine in a light emitting layer to cause emission. What is important here is how efficiently the hole and electron charges are transferred to the light emitting layer in order to form a device having excellent carrier balance. The probability of hole-electron recombination can be improved by improving hole injectability and electron blocking performance of blocking injected electrons from the cathode, and high luminous efficiency can be obtained by confining excitons generated in the light emitting layer. The role of a hole transport material is therefore important, and there is a need for a hole transport material that has high hole injectability, high hole mobility, high electron blocking performance, and high durability to electrons.

Heat resistance and amorphousness of the materials are also important with respect to the lifetime of the device. The materials with low heat resistance cause thermal decomposition even at a low temperature by heat generated during the drive of the device, which leads to the deterioration of the materials. The materials with low amorphousness cause crystallization of a thin film even in a short time and lead to the deterioration of the device. The materials in use are therefore required to have characteristics of high heat resistance and satisfactory amorphousness.

N,N'-diphenyl-N,N'-di(α-naphthyl)benzidine (NPD) and various aromatic amine derivatives are known as the hole transport materials used for the organic EL device (refer to Patent Documents 1 and 2, for example). Although NPD has desirable hole transportability, its glass transition point (Tg), which is an index of heat resistance, is as low as 96°C, which causes the degradation of device characteristics by crystallization under a hightemperature condition (refer to Non-Patent Document 4, for example). The aromatic amine derivatives described in the Patent Documents include a compound known to have an excellent hole mobility of 10⁻³ cm²/Vs or higher (refer to Patent Documents 1 and 2, for example). However, since the compound is insufficient in terms of electron blocking performance, some of the electrons pass through the light emitting layer, and improvements in luminous efficiency cannot be expected. For such a reason, a material with higher electron blocking performance, a more stable thin-film state and higher heat resistance is needed for higher efficiency. Although an aromatic amine derivative having high durability is reported (refer to Patent Document 3, for example), the derivative is used as a charge transporting material used in an electrophotographic photoconductor, and there is no example of using the derivative in the organic EL device.

Arylamine compounds having a substituted carbazole structure are proposed as compounds improved in the characteristics such as heat resistance and hole injectability (refer to Patent Documents 4 and 5, for example). However, while the devices using these compounds for the hole injection layer or the hole transport layer have been improved in heat resistance, luminous efficiency and the like, the improvements are still insufficient. Further lower driving voltage and higher luminous efficiency are therefore needed. Patent Document 7 describes a compound having a triarylamine structure and an electroluminescence device. Patent Document 8 describes a heterocyclic compound, and an organic light-emitting device comprising the same. Patent Document 9 describes a polycyclic aromatic compound, and an organic electroluminescent (EL) element, an organic field effect transistor and an organic thin film solar cell using the polycyclic aromatic compound, as well as a display apparatus and a lighting apparatus.

In order to improve characteristics of the organic EL device and to improve the yield of the device production, it has been desired to develop a device having high luminous efficiency, low driving voltage and a long lifetime by using in combination the materials that excel in hole and electron injection/transport performances, stability as a thin film and durability, permitting holes and electrons to be highly efficiently recombined together.

Further, in order to improve characteristics of the organic EL device, it has been desired to develop a device that maintains carrier balance and has high efficiency, low driving voltage and a long lifetime by using in combination the materials that excel in hole and electron injection/transport performances, stability as a thin film and durability.

### Citation List

### Patent Documents

Patent Document 1: JP-A-Hei-8-048656
Patent Document 2: Japanese Patent No. 3194657
Patent Document 3: Japanese Patent No. 4943840
Patent Document 4: JP-A-2006-151979
Patent Document 5: WO2008/062636
Patent Document 6: WO2014/009310
Patent Document 7: WO 2020/004235 A1
Patent Document 8: WO 2019/164331 A1
Patent Document 9: US 2015/236274 A1

### Non-Patent Documents

Non-Patent Document 1: The Japan Society of Applied Physics, 9th Lecture Preprints, pp. 55 to 61 (2001)
Non-Patent Document 2: The Japan Society of Applied Physics, 9th Lecture Preprints, pp. 23 to 31 (2001)
Non-Patent Document 3: Appl. Phys. Let., 98, 083302 (2011)
Non-Patent Document 4: Organic EL Symposium, the 3rd Regular presentation Preprints, pp. 13 to 14 (2006)

### Summary of the Invention

### Technical Problem

An object of the present invention is to provide materials for an organic EL device with excellent hole injection and transport performance, electron blocking capability, stability and durability in the thin-film state, and also to provide an organic EL device having a high efficiency, a low driving voltage, and a long lifetime by combining the material with various materials for an organic EL device that is excellent in hole and electron injection and transport abilities, electron blocking ability, stability and durability in the thin-film state, in such a manner that the characteristics of the materials can be effectively exhibited.

Physical properties of the organic compound to be provided by the present invention include (1) good hole injection characteristics, (2) large hole mobility, (3) excellent electron blocking ability, (4) stability in a thin-film state, and (5) excellent heat resistance.

Physical properties of the organic EL device to be provided by the present invention include (1) high luminous efficiency and high power efficiency, (2) low turn on voltage, (3) low actual driving voltage, and (4) a long lifetime.

### Solution to Problem

For achieving the object, the present inventors have focused the fact that a triarylamine material having a specific structure is excellent in hole injection / transport ability, thin film stability, and durability. The various triarylamine compounds of the above were diligently investigated and the characteristics of the prepared an organic EL device were evaluated diligently. As a result, the present inventors have found that when a triarylamine compound having a specific structure is used as a material for the hole transport layer, holes injected from the anode side can be efficiently transported. Furthermore, various organic EL devices combining light-emitting materials having a specific structure and the like were manufactured, and the characteristics of the devices were evaluated diligently. As a result, they have completed the present invention.

Specifically, according to the present invention, the following organic EL devices are provided.

1) An organic EL device comprising at least a first hole transport layer, a second hole transport layer, a blue light emitting layer and an electron transport layer in this order from the anode side between anode and cathode, wherein the second hole transport layer, or at least one layer of the laminated layers disposed between the first hole transport layer and the electron transport layer comprises a triarylamine compound represented by the following general formula (1):

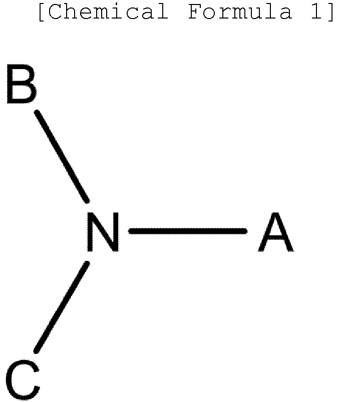

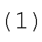

In the formula, A, B, and C may be the same or different, and represent a monovalent group of the general formula (2-1), where the dashed part is the bonding site, a substituted or unsubstituted aromatic hydrocarbon group, a substituted or unsubstituted aromatic heterocyclic group, or a substituted or unsubstituted condensed polycyclic aromatic group. However, all of A, B and C shall not simultaneously be monovalent groups represented by the following general formula (2-1).

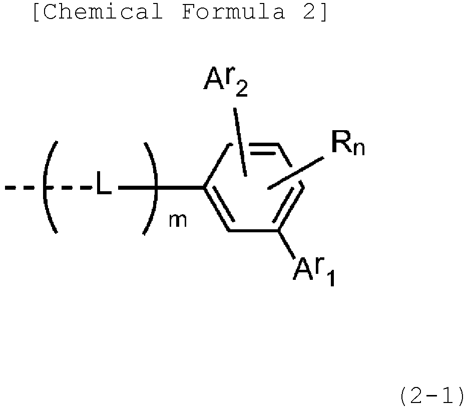

In the formula, the dashed part is the binding site. R represents a deuterium atom, a fluorine atom, a chlorine atom, a cyano group, a nitro group, a linear or branched alkyl group of 1 to 6 carbon atoms that may have a substituent, a cycloalkyl group of 5 to 10 carbon atoms that may have a substituent, a linear or branched alkenyl group of 2 to 6 carbon atoms that may have a substituent, a linear or branched alkyloxy group of 1 to 6 carbon atoms that may have a substituent, a cycloalkyloxy group of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or a substituted or unsubstituted aryloxy group. n is the number of R and represents an integer between 0 and 3. When n is 2 or 3, a plurality of R which bind to the same benzene ring, may be the same or different and may bind to each other via a single bond, substituted or unsubstituted methylene group, an oxygen atom, or a sulfur atom to form a ring. L represents a divalent group of a substituted or unsubstituted aromatic hydrocarbon, a divalent group of a substituted or unsubstituted aromatic heterocyclic ring, or a divalent group of substituted or unsubstituted condensed polycyclic aromatics. m represents an integer between 1 and 3. When m is 2 or 3, L may be the same or different. Ar₁ and Ar₂ may be the same or different, and represent a substituted or unsubstituted aromatic hydrocarbon group, a substituted or unsubstituted aromatic heterocyclic group, or a substituted or unsubstituted condensed polycyclic aromatic group, wherein the blue light emitting layer includes a blue light emitting dopant, and
wherein the blue light emitting dopant is a compound represented by the following general formula (3-1) or general formula (3-2) 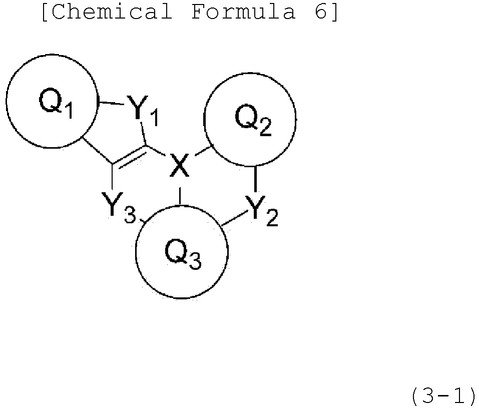 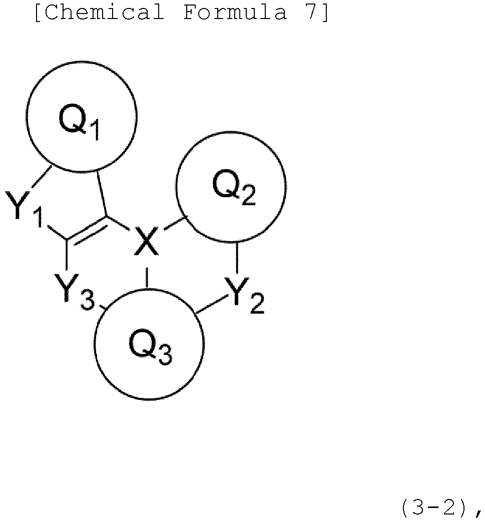
wherein Q₁ to Q₃ may be the same or different, and represent a substituted or unsubstituted aromatic hydrocarbon, a substituted or unsubstituted condensed polycyclic aromatics, or a substituted or unsubstituted aromatic heterocyclic ring, X represents B, P, P = O, or P = S, Y₁ to Y₃ may be the same or different, and represent one of selected from N-R₄, CR₅R₆, O, S, Se and SiR₇R_{B}, R₄ to R₈ may be the same or different, and represent a hydrogen atom, a deuterium atom, a fluorine atom, a chlorine atom, a cyano group, a nitro group, a linear or branched alkyl group of 1 to 6 carbon atoms that may have a substituent, a cycloalkyl group of 5 to 10 carbon atoms that may have a substituent, a linear or branched alkenyl group of 2 to 6 carbon atoms that may have a substituent, a linear or branched alkyloxy group of 1 to 6 carbon atoms that may have a substituent, a cycloalkyloxy group of 5 to 10 carbon atoms that may have a substituent, a substituted or unsubstituted aromatic hydrocarbon group, a substituted or unsubstituted condensed polycyclic aromatic group, or a substituted or unsubstituted aryloxy group, R₅ and R₆, and R₇ and R₈ may bind to each other to form a ring via a single bond, substituted or unsubstituted methylene group, an oxygen atom, or a sulfur atom, when Y₁ to Y₃ are N-R₄, CR₅R₆, or SiR₇R_{B}, R₄ to R₈ may bind to the adjacent Q₁, Q₂, or Q₃, respectively, to form a ring via a linking group, such as substituted or unsubstituted methylene group, an oxygen atom, a sulfur atom, or a monosubstituted amino group, and
wherein the aromatic hydrocarbon, the condensed polycyclic aromatics, and the aromatic heterocyclic ring in the substituted or unsubstituted aromatic hydrocarbon, the substituted or unsubstituted condensed polycyclic aromatics, and the substituted or unsubstituted aromatic heterocyclic ring represented by Q₁ to Q₃ in the general formula (3-1) and the general formula (3-2) are selected from the group consisting of benzene, naphthalene, anthracene, fluorene, phenanthrene, pyridine, pyrimidine, triazine, pyrrole, quinoline, isoquinoline, indene, indole, indoline, carbazole, carboline, benzoxazole, benzothiazole, quinoxaline, benzimidazole, pyrazole, dibenzofuran, dibenzothiophene, naphthyridine, phenanthroline, and acridine.

2) The organic EL device of 1), wherein the monovalent group represented by the general formula (2-1) is the monovalent group represented by the following general formula (2-2):

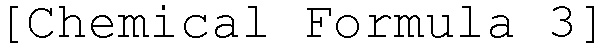

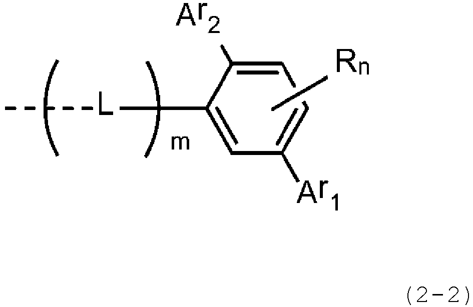

In the formula, the dashed part is the binding site. Ar₁, Ar₂, L, m, n and R are the same as defined by the general formula (2-1).

3) The organic EL device of 1), wherein the monovalent group represented by the general formula (2-1) is the monovalent group represented by the following general formula (2-3):

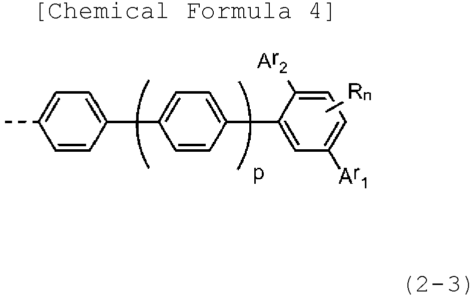

In the formula, the dashed part is the binding site. Ar₁, Ar₂, n and R are the same as defined by the general formula (2-1). p represents 0 or 1.

4) The organic EL device of 1), wherein the monovalent group represented by the general formula (2-1) is the monovalent group represented by the following general formula (2-4):

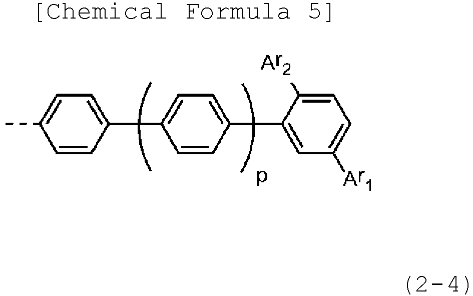

In the formula, the dashed part is the binding site. Ar₁, and Ar₂ are the same as defined by the general formula (2-1). p represents 0 or 1.

5) The organic EL device of any one of 1) to 4), wherein the blue light emitting layer includes an anthracene derivative having an anthracene skeleton in the molecule.
6) The organic EL device of 5), wherein the blue light emitting layer includes a host material that is an anthracene derivative having an anthracene skeleton in the molecule.

Specific examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by R in the general formula (2-1) include phenyl, biphenylyl, terphenylyl, naphthyl, anthracenyl, phenanthrenyl, fluorenyl, spirobifluorenyl, indenyl, pyrenyl, perylenyl, fluoranthenyl, triphenylenyl, pyridyl, pyrimidinyl, triazinyl, furyl, pyrrolyl, thienyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, naphthyridinyl, phenanthrolinyl, acridinyl, carbolinyl, or the like, and an aryl group consisting of 6 to 30 carbon atoms, or a heteroaryl group consisting of 2 to 30 carbon atoms can be selected.

When more than one of these groups is bonded to the same benzene ring, they may be bonded to each other via a single bond, a substituted or unsubstituted methylene group, an oxygen atom or a sulfur atom to form a ring, or they may be bonded to the benzene ring to which each group is attached via a single bond, a substituted or unsubstituted methylene group, an oxygen atom or a sulfur atom to form a ring.

Specific examples of the "substituent" in the "substituted aromatic hydrocarbon group", the "substituted aromatic heterocyclic group", or the "substituted condensed polycyclic aromatic group" represented by R in the general formula (2-1) include a deuterium atom, cyano, nitro; halogen atoms such as a fluorine atom, a chlorine atom, a bromine atom, and an iodine atom; silyl group such as trimethylsilyl and triphenylsilyl; linear or branched alkyl group of 1 to 6 carbon atoms such as methyl, ethyl, and propyl; linear or branched alkyloxy group of 1 to 6 carbon atoms such as methyloxy, ethyloxy, and propyloxy; alkenyl group such as vinyl and allyl; aryloxy group such as phenyloxy and tolyloxy; arylalkyloxy group such as benzyloxy and phenethyloxy; aromatic hydrocarbon groups or condensed polycyclic aromatic groups such as phenyl, biphenylyl, terphenylyl, naphthyl, anthracenyl, phenanthrenyl, fluorenyl, spirobifluorenyl, indenyl, pyrenyl, perylenyl, fluoranthenyl, and triphenylenyl; aromatic heterocyclic groups such as pyridyl, thienyl, furyl, pyrrolyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, and carbolinyl. These substituents may be further substituted with the exemplified substituents above.

These substituents may bind to each other via a single bond, substituted or unsubstituted methylene group, substituted or unsubstituted amino group, an oxygen atom, or a sulfur atom to form a ring.

Specific examples of the "linear or branched alkyl group of 1 to 6 carbon atoms", the "cycloalkyl group of 5 to 10 carbon atoms", or the "linear or branched alkenyl group of 2 to 6 carbon atoms" in the "linear or branched alkyl group of 1 to 6 carbon atoms that may have a substituent", the "cycloalkyl group of 5 to 10 carbon atoms that may have a substituent", or the "linear or branched alkenyl group of 2 to 6 carbon atoms that may have a substituent" represented by R in the general formula (2-1) include a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamantyl group, a 2-adamantyl group, a vinyl group, an allyl group, an isopropenyl group, and a 2-butenyl group.

When more than one of these groups is bonded to the same benzene ring, they may be bonded to each other via a single bond, a substituted or unsubstituted methylene group, an oxygen atom or a sulfur atom to form a ring, or they may be bonded to the benzene ring to which each group is attached via a single bond, a substituted or unsubstituted methylene group, an oxygen atom or a sulfur atom to form a ring.

Specific examples of the "substituent" in the "linear or branched alkyl of 1 to 6 carbon atoms having a substituent", the "cycloalkyl of 5 to 10 carbon atoms having a substituent", or the "linear or branched alkenyl of 2 to 6 carbon atoms having a substituent" represented by R in the general formula (2-1) include a deuterium atom, cyano, nitro; halogen atoms such as a fluorine atom, a chlorine atom, a bromine atom, and an iodine atom; silyl group such as trimethylsilyl and triphenylsilyl; linear or branched alkyloxy group of 1 to 6 carbon atoms such as methyloxy, ethyloxy, and propyloxy; alkenyl group such as vinyl and allyl; aryloxy group such as phenyloxy and tolyloxy; arylalkyloxy group such as benzyloxy and phenethyloxy; aromatic hydrocarbon groups or condensed polycyclic aromatic groups such as phenyl, biphenylyl, terphenylyl, naphthyl, anthracenyl, phenanthrenyl, fluorenyl, spirobifluorenyl, indenyl, pyrenyl, perylenyl, fluoranthenyl, and triphenylenyl; aromatic heterocyclic groups such as pyridyl, thienyl, furyl, pyrrolyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, carbolinyl. These substituents may be further substituted with the exemplified substituents above.

These substituents may bind to each other via a single bond, substituted or unsubstituted methylene group, substituted or unsubstituted amino group, an oxygen atom, or a sulfur atom to form a ring.

Specific examples of the "linear or branched alkyloxy group of 1 to 6 carbon atoms" or the "cycloalkyloxy group of 5 to 10 carbon atoms" in the "linear or branched alkyloxy group of 1 to 6 carbon atoms that may have a substituent" or the "cycloalkyloxy group of 5 to 10 carbon atoms that may have a substituent" represented by R in the general formula (2-1) include a methyloxy group, an ethyloxy group, an n-propyloxy group, an isopropyloxy group, an n-butyloxy group, a tert-butyloxy group, an n-pentyloxy group, an n-hexyloxy group, a cyclopentyloxy group, a cyclohexyloxy group, a cycloheptyloxy group, a cyclooctyloxy group, a 1-adamantyloxy group, and a 2-adamantyloxy group.

When more than one of these groups is bonded to the same benzene ring, they may be bonded to each other via a single bond, a substituted or unsubstituted methylene group, an oxygen atom or a sulfur atom to form a ring, or they may be bonded to the benzene ring to which each group is attached via a single bond, a substituted or unsubstituted methylene group, an oxygen atom or a sulfur atom to form a ring.

Specific examples of the "substituent" in the "linear or branched alkyloxy group of 1 to 6 carbon atoms having a substituent", or the "cycloalkyloxy group of 5 to 10 carbon atoms having a substituent" represented by R in the general formula (2-1) include a deuterium atom, cyano, nitro; halogen atoms such as a fluorine atom, a chlorine atom, a bromine atom, and an iodine atom; silyl group such as trimethylsilyl and triphenylsilyl; linear or branched alkyloxy group of 1 to 6 carbon atoms such as methyloxy, ethyloxy, and propyloxy; alkenyl group such as vinyl and allyl; aryloxy group such as phenyloxy and tolyloxy; arylalkyloxy group such as benzyloxy and phenethyloxy; aromatic hydrocarbon groups or condensed polycyclic aromatic groups such as phenyl, biphenylyl, terphenylyl, naphthyl, anthracenyl, phenanthrenyl, fluorenyl, spirobifluorenyl, indenyl, pyrenyl, perylenyl, fluoranthenyl, and triphenylenyl; aromatic heterocyclic groups such as pyridyl, thienyl, furyl, pyrrolyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, carbolinyl. These substituents may be further substituted with the exemplified substituents above.

These substituents may bind to each other via a single bond, substituted or unsubstituted methylene group, substituted or unsubstituted amino group, an oxygen atom, or a sulfur atom to form a ring.

Specific examples of the "aryloxy group" in the "substituted or unsubstituted aryloxy group" represented by R in the general formula (2-1) include a phenyloxy group, a biphenylyloxy group, a terphenylyloxy group, a naphthyloxy group, an anthracenyloxy group, a phenanthrenyloxy group, a fluorenyloxy group, an indenyloxy group, a pyrenyloxy group, and a perylenyloxy group.

When more than one of these groups is bonded to the same benzene ring, they may be bonded to each other via a single bond, a substituted or unsubstituted methylene group, an oxygen atom or a sulfur atom to form a ring, or they may be bonded to the benzene ring to which each group is attached via a single bond, a substituted or unsubstituted methylene group, an oxygen atom or a sulfur atom to form a ring.

Examples of the "substituent" in the "substituted aryloxy group" represented by R in the general formula (2-1) include the same substituents exemplified as the "substituent" in the "substituted aromatic hydrocarbon group", the "substituted aromatic heterocyclic group", or the "substituted condensed polycyclic aromatic group" represented by R in the general formula (2-1), and these substituents may bind to each other via a single bond, substituted or unsubstituted methylene group, substituted or unsubstituted amino group, an oxygen atom, or a sulfur atom to form a ring.

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ and Ar₂ in the general formula (2-1) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by R in the general formula (2-1).

Examples of the "substituent" in the "substituted aromatic hydrocarbon group", the "substituted aromatic heterocyclic group", or the "substituted condensed polycyclic aromatic group" represented by Ar₁ and Ar₂ in the general formula (2-1) include the same substituents exemplified as the "substituent" in the "substituted aromatic hydrocarbon group", the "substituted aromatic heterocyclic group", or the "substituted condensed polycyclic aromatic group" represented by R in the general formula (2-1), and these substituents may bind to each other via a single bond, substituted or unsubstituted methylene group, substituted or unsubstituted amino group, an oxygen atom, or a sulfur atom to form a ring.

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by A, B and C in the general formula (1) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by R in the general formula (2-1).

Examples of the "substituent" in the "substituted aromatic hydrocarbon group", the "substituted aromatic heterocyclic group", or the "substituted condensed polycyclic aromatic group" represented by A, B and C in the general formula (1) include the same substituents exemplified as the "substituent" in the "substituted aromatic hydrocarbon group", the "substituted aromatic heterocyclic group", or the "substituted condensed polycyclic aromatic group" represented by R in the general formula (2-1), and these substituents may bind to each other via a single bond, substituted or unsubstituted methylene group, substituted or unsubstituted amino group, an oxygen atom, or a sulfur atom to form a ring.

Specific examples of the "aromatic hydrocarbon", the "aromatic heterocyclic ring", or the "condensed polycyclic aromatics" of the "substituted or unsubstituted aromatic hydrocarbon", the "substituted or unsubstituted aromatic heterocyclic ring", or the "substituted or unsubstituted condensed polycyclic aromatics" in the "divalent group of a substituted or unsubstituted aromatic hydrocarbon", the "divalent group of a substituted or unsubstituted aromatic heterocyclic ring", or the "divalent group of substituted or unsubstituted condensed polycyclic aromatics" represented by L in the general formula (2-1) include benzene, biphenyl, terphenyl, tetrakisphenyl, styrene, naphthalene, anthracene, acenaphthalene, fluorene, phenanthrene, indane, pyrene, triphenylene, pyridine, pyrimidine, triazine, pyrrole, furan, thiophene, quinoline, isoquinoline, benzofuran, benzothiophene, indoline, carbazole, carboline, benzoxazole, benzothiazole, quinoxaline, benzimidazole, pyrazole, dibenzofuran, dibenzothiophene, naphthyridine, phenanthroline, and acridine.

The "divalent group of an aromatic hydrocarbon", the "divalent group of an aromatic heterocyclic ring", or the "divalent group of condensed polycyclic aromatics" in the "divalent group of a substituted or unsubstituted aromatic hydrocarbon", the "divalent group of a substituted or unsubstituted aromatic heterocyclic ring", or the "divalent group of substituted or unsubstituted condensed polycyclic aromatics" represented by L in the general formula (2-1) is a divalent group that results from the removal of two hydrogen atoms from the above "aromatic hydrocarbon", "aromatic heterocyclic ring", or "condensed polycyclic aromatics".

Examples of the "substituent" in the "divalent group of a substituted aromatic hydrocarbon", the "divalent group of a substituted aromatic heterocyclic ring", or the "divalent group of substituted condensed polycyclic aromatics" represented by L in the general formula (2-1) include the same substituents exemplified as the "substituent" in the "substituted aromatic hydrocarbon group", the "substituted aromatic heterocyclic group", or the "substituted condensed polycyclic aromatic group" represented by R in the general formula (2-1), and these substituents may bind to each other via a single bond, substituted or unsubstituted methylene group, substituted or unsubstituted amino group, an oxygen atom, or a sulfur atom to form a ring.

In the triarylamine compounds represented by general formula (1), it is preferred that at least one of A, B and C is a monovalent group represented by the general formula (2-1).

In the triarylamine compounds represented by general formula (1), the monovalent group represented by the general formula (2-1) is preferably a monovalent group represented by the general formula (2-2), far preferably, a monovalent group represented by the general formula (2-3), further preferably a monovalent group represented by the general formula (2-4).

In the triarylamine compounds represented by general formula (1), Ar₁ and/or Ar₂ in the general formula (2-1) to (2-4) is preferably the "substituted or unsubstituted aromatic hydrocarbon", or the "substituted or unsubstituted condensed polycyclic aromatic group", far preferably, a substituted or unsubstituted phenyl group, a substituted or unsubstituted naphthyl group, or a substituted or unsubstituted biphenyl group, further preferably an unsubstituted phenyl group, or an unsubstituted naphthyl group.

In the triarylamine compounds represented by general formula (1), L in the general formula (2-1) to (2-2) is preferably the "divalent group of an unsubstituted aromatic hydrocarbon", "divalent group of an unsubstituted aromatic heterocyclic ring", or the "divalent group of an unsubstituted condensed polycyclic aromatic group". Further, it is preferably a divalent group that results from the removal of two hydrogen atoms from the "aromatic hydrocarbon", or "condensed polycyclic aromatics", far preferably a divalent group that results from the removal of two hydrogen atoms from benzene (phenylene group).

In the triarylamine compounds represented by general formula (1), the number n of substituents R in general formula (2-1) is preferably zero (0), i.e. no substituent R.

In the triarylamine compounds represented by general formula (1), the number m of a divalent group L in general formula (2-1) is preferably 1 or 2.

The "aromatic hydrocarbon", the "condensed polycyclic aromatics", and the "aromatic heterocyclic ring" in the "substituted or unsubstituted aromatic hydrocarbon", the "substituted or unsubstituted condensed polycyclic aromatics", and the "substituted or unsubstituted aromatic heterocyclic ring" represented by Q₁ to Q₃ in the general formula (3-1) and the general formula (3-2) are selected from the group consisting of benzene, naphthalene, anthracene, fluorene, phenanthrene, pyridine, pyrimidine, triazine, pyrrole, quinoline, isoquinoline, indene, indole, indoline, carbazole, carboline, benzoxazole, benzothiazole, quinoxaline, benzimidazole, pyrazole, dibenzofuran, dibenzothiophene, naphthyridine, phenanthroline, and acridine.

These may have a substituent, and examples of the substituent include the same substituents exemplified as the "substituent" in the "substituted aromatic hydrocarbon group", the "substituted aromatic heterocyclic group", or the "substituted condensed polycyclic aromatic group" represented by R in the general formula (2-1). These substituents may bind to each other via a single bond, substituted or unsubstituted methylene group, an oxygen atom, or a sulfur atom to form a ring.

X in the general formula (3-1) and the general formula (3-2) represents B, P, P = O, or P = S. B is defined as a boron atom, P is a phosphorus atom, P = O is a phosphorus atom in which an oxygen atom is bonded by a double bond, and P = S is defined as a phosphorus atom in which a sulfur atom is bonded by a double bond.

Y₁ to Y₃ in the general formula (3-1) and the general formula (3-2) may be the same or different, and represent one of selected from N-R₄, CR₃R₆, O, S, Se, and SiR₇R₈. N-R₄ is a nitrogen atom having R₄ as a substituent, CR₅R₆ is a carbon atom having R₅ and R₅ as a substituent, O is an oxygen atom, S is a sulfur atom, Se is a selenium atom, and SiR₇R₈ is a silicon atom having R₇ and R₈ as a substituent.

In this case, R₄ to R₈ may bind to the adjacent Q₁, Q₂, or Q₃, i.e., Q₁ if Y₁ is N-R₄,CR₅R₆ or SiR₇R₈, Q₂ or Q₃ if Y₂ is N-R₄, CR₅R₆ or SiR₇R_{B}, Q₃ if Y₃ is N-R₄, CR₅R₆ or SiR₇R_{B}, respectively, to form a ring via a linking group, such as substituted or unsubstituted methylene group, an oxygen atom, a sulfur atom, or a monosubstituted amino group.

Furthermore, R₅ and R₆, R₇ and R₈ may bind to each other via a single bond, substituted or unsubstituted methylene group, an oxygen atom, or a sulfur atom to form a ring.

The definitions of R4 to R8 will be further described in detail below.

When Y₁ to Y₃ in the general formula (3-1) and the general formula (3-2) are N-R₄, CR₅R₆, or SiR₇R_{B}, specific examples of the "linear or branched alkyl group of 1 to 6 carbon atoms", the "cycloalkyl group of 5 to 10 carbon atoms", or the "linear or branched alkenyl group of 2 to 6 carbon atoms" in the "linear or branched alkyl group of 1 to 6 carbon atoms that may have a substituent", the "cycloalkyl group of 5 to 10 carbon atoms that may have a substituent", or the "linear or branched alkenyl group of 2 to 6 carbon atoms that may have a substituent" represented by R₄ to R₈ in N-R₄, CR₅R₆, or SiR₇R₈ include a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamantyl group, a 2-adamantyl group, a vinyl group, an allyl group, an isopropenyl group, and a 2-butenyl group.

These groups may have a substituent, and examples of the substituent include the same substituents exemplified as the "substituent" in the "linear or branched alkyl of 1 to 6 carbon atoms having a substituent", the "cycloalkyl of 5 to 10 carbon atoms having a substituent", or the "linear or branched alkenyl of 2 to 6 carbon atoms having a substituent" represented by R in the general formula (2-1).

When Y₁ to Y₃ in the general formula (3-1) and the general formula (3-2) are N-R₄, CR₅R₆, or SiR₇R_{B}, specific examples of the "linear or branched alkyloxy group of 1 to 6 carbon atoms" or the "cycloalkyloxy group of 5 to 10 carbon atoms" in the "linear or branched alkyloxy group of 1 to 6 carbon atoms that may have a substituent" or the "cycloalkyloxy group of 5 to 10 carbon atoms that may have a substituent" represented by R₄ to R₈ in N-R₄, CR₅R₆, or SiR₇R₈ include a methyloxy group, an ethyloxy group, an n-propyloxy group, an isopropyloxy group, an n-butyloxy group, a tert-butyloxy group, an n-pentyloxy group, an n-hexyloxy group, a cyclopentyloxy group, a cyclohexyloxy group, a cycloheptyloxy group, a cyclooctyloxy group, a 1-adamantyloxy group, and a 2-adamantyloxy group.

These groups may have a substituent, and examples of the substituent include the same substituents exemplified as the "substituent" in the "linear or branched alkyloxy group of 1 to 6 carbon atoms having a substituent", or the "cycloalkyloxy group of 5 to 10 carbon atoms having a substituent" represented by R in the general formula (2-1).

When Y₁ to Y₃ in the general formula (3-1) and the general formula (3-2) are N-R₄, CR₅R₆, or SiR₇R_{B}, specific examples of the "aromatic hydrocarbon group", or the "condensed polycyclic aromatic group" in the "substituted or unsubstituted aromatic hydrocarbon group", or the "substituted or unsubstituted condensed polycyclic aromatic group" represented by R₄ to R₈ in N-R₄, CR₅R₆, or SiR₇R₈ include phenyl, biphenyl, terphenyl, naphthyl, anthracenyl, phenanthryl.

These groups may have a substituent, and examples of the substituent include the same substituents exemplified as the "substituent" in the "substituted aromatic hydrocarbon group", the "substituted aromatic heterocyclic group", or the "substituted condensed polycyclic aromatic group" represented by R in the general formula (2-1).

When Y₁ to Y₃ in the general formula (3-1) and the general formula (3-2) are N-R₄, CR₅R₆, or SiR₇R_{B}, specific examples of the "aryloxy group" in the "substituted or unsubstituted aryloxy group" represented by R₄ to R₈ in N-R₄, CR₅R₆, or SiR₇R₈ include a phenyloxy group, a biphenylyloxy group, a terphenylyloxy group, a naphthyloxy group, an anthracenyloxy group, a phenanthrenyloxy group, a fluorenyloxy group, an indenyloxy group, a pyrenyloxy group, and a perylenyloxy group.

These groups may have a substituent, and examples of the substituent include the same substituents exemplified as the "substituent" in the "substituted aromatic hydrocarbon group", the "substituted aromatic heterocyclic group", or the "substituted condensed polycyclic aromatic group" represented by R in the general formula (2-1).

In the general formulas (3-1) and the general formula (3-2), the "aromatic hydrocarbon", the "condensed polycyclic aromatics", or the "aromatic heterocyclic ring" in the "substituted or unsubstituted aromatic hydrocarbon", the " substituted or unsubstituted condensed polycyclic aromatics", or the "substituted or unsubstituted aromatic heterocyclic ring" represented by Q₁ to Q₃ are preferably benzene, naphthalene, phenanthrene, pyridine, pyrimidine, indene, and indol, far preferably, benzene and naphthalene.

In the general formula (3-1) and the general formula (3-2), when Y₁ to Y₃ are N-R₄, CR₅R₆, or SiR₇R_{B}, R₄ to R₈ in N-R₄, CR₅R₆, or SiR₇R₈ are preferably a "linear or branched alkyl group of 1 to 6 carbon atoms that may have a substituent", a "cycloalkyl group of 5 to 10 carbon atoms that may have a substituent", a "linear or branched alkenyl group of 2 to 6 carbon atoms that may have a substituent", a "linear or branched alkyloxy group of 1 to 6 carbon atoms that may have a substituent", a "cycloalkyloxy group of 5 to 10 carbon atoms that may have a substituent", a "substituted or unsubstituted aromatic hydrocarbon group", a "substituted or unsubstituted condensed polycyclic aromatic group", or a "substituted or unsubstituted aryloxy group", R₄ in N-R₄ is far preferably a "linear or branched alkyl group of 1 to 6 carbon atoms that may have a substituent", a "cycloalkyl group of 5 to 10 carbon atoms that may have a substituent", a "linear or branched alkenyl group of 2 to 6 carbon atoms that may have a substituent", a "substituted or unsubstituted aromatic hydrocarbon group", or a "substituted or unsubstituted condensed polycyclic aromatic group".

In the general formula (3-1) and the general formula (3-2), Y₁ is preferably N-R₄, O, or S, far preferably, O, or S.

In the general formula (3-1) and the general formula (3-2), it is preferable that at least one of Y₂ and Y₃ is N-R₄, far preferably, both of Y₂ and Y₃ is N-R₄. R₄ in N-R₄ is preferably a "substituted or unsubstituted aromatic hydrocarbon group", or a "substituted or unsubstituted condensed polycyclic aromatic group", far preferably, a substituted or unsubstituted phenyl group, a substituted or unsubstituted biphenyl group, a substituted or unsubstituted terphenyl group, or a substituted or unsubstituted naphthyl group.

### Effects of the Invention

The triarylamine compound represented by the general formula (1) of the present invention has (1) good hole injection characteristics, (2) large hole mobility, (3) excellent electron blocking ability, (4) stability in a thin-film state, and (5) excellent heat resistance, and is therefore preferably used as a constituent material of the hole transport layer of the organic EL element of the present invention.

The organic EL device of the present invention uses a triarylamine compound represented by the general formula (1) as a constituent material of the hole transport layer. Therefore, the high efficiency, low turn on voltage, and high durability of the organic EL device in the present invention can be achieved because of the use of the triarylamine compound, which has greater hole mobility, superior electron blocking ability, superior amorphousness, and a more stable thin-film state than conventional hole transport materials.

Further, in the present invention, the hole transport layer has a two-layer structure of a first hole transport layer and a second hole transport layer, and the second hole transport layer located on the light emitting layer side includes the triarylamine compound of the general formula (1), and thus, the electron blocking performance of the triarylamine compound can be fully utilized. Thus, a more efficient, and high durability of the organic EL device can be realized.

In the present invention, the second hole transport layer, or at least one layer of the laminated layers disposed between the first hole transport layer and the electron transport layer comprises a triarylamine compound represented by the general formula (1), the high efficiency, low turn on voltage, and high durability of the organic EL device in the present invention can be achieved because of the use of the triarylamine compound, which has superior amorphousness, and a more stable thin-film state than conventional hole transport materials.

### Brief Description of the Drawings

Fig. 1 is a figure showing the structural formula of the compound 1-1 to 1-12 as examples of triarylamine compound represented by the general formula (1).
Fig. 2 is a figure showing the structural formula of the compound 1-13 to 1-24 as examples of triarylamine compound represented by the general formula (1).
Fig. 3 is a figure showing the structural formula of the compound 1-25 to 1-36 as examples of triarylamine compound represented by the general formula (1).
Fig. 4 is a figure showing the structural formula of the compound 1-37 to 1-48 as examples of triarylamine compound represented by the general formula (1).
Fig. 5 is a figure showing the structural formula of the compound 1-49 to 1-60 as examples of triarylamine compound represented by the general formula (1).
Fig. 6 is a figure showing the structural formula of the compound 1-61 to 1-72 as examples of triarylamine compound represented by the general formula (1).
Fig. 7 is a figure showing the structural formula of the compound 1-73 to 1-84 as examples of triarylamine compound represented by the general formula (1).
Fig. 8 is a figure showing the structural formula of the compound 1-85 to 1-95 as examples of triarylamine compound represented by the general formula (1).
Fig. 9 is a figure showing the structural formula of the compound 1-96 to 1-107 as examples of triarylamine compound represented by the general formula (1).
Fig. 10 is a figure showing the structural formula of the compound 1-108 to 1-119 as examples of triarylamine compound represented by the general formula (1).
Fig. 11 is a figure showing the structural formula of the compound 1-120 to 1-131 as examples of triarylamine compound represented by the general formula (1).
Fig. 12 is a figure showing the structural formula of the compound 1-132 to 1-142 as examples of triarylamine compound represented by the general formula (1).
Fig. 13 is a figure showing the structural formula of the compound 1-143 to 1-154 as examples of triarylamine compound represented by the general formula (1).
Fig. 14 is a figure showing the structural formula of the compound 1-155 to 1-166 as examples of triarylamine compound represented by the general formula (1).
Fig. 15 is a figure showing the structural formula of the compound 1-167 to 1-178 as examples of triarylamine compound represented by the general formula (1).
Fig. 16 is a figure showing the structural formula of the compound 1-179 to 1-190 as examples of triarylamine compound represented by the general formula (1).
Fig. 17 is a figure showing the structural formula of the compound 1-191 to 1-201 as examples of triarylamine compound represented by the general formula (1).
Fig. 18 is a figure showing the structural formula of the compound 3-1-1 to 3-1-9 as examples of compound represented by the general formula (3-1).
Fig. 19 is a figure showing the structural formula of the compound 3-1-10 to 3-1-21, 3-1-23 and 3-1-24 as examples of compound represented by the general formula (3-1).
Fig. 20 is a figure showing the structural formula of the compound 3-2-1 to 3-2-6 as examples of compound represented by the general formula (3-2).
Fig. 21 is a diagram illustrating as examples of the configuration of the organic EL devices in the present invention.

### Mode for Carrying Out the Invention

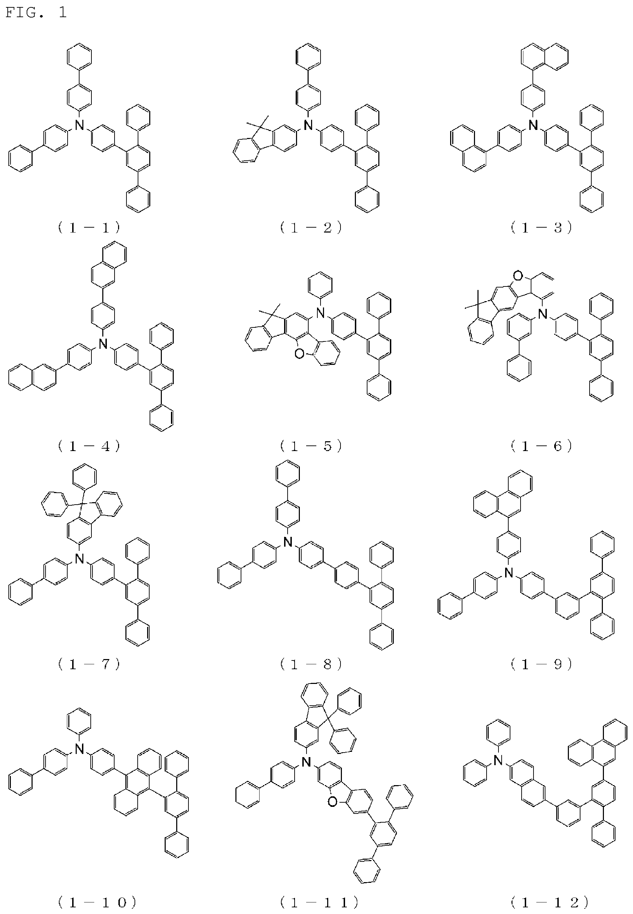

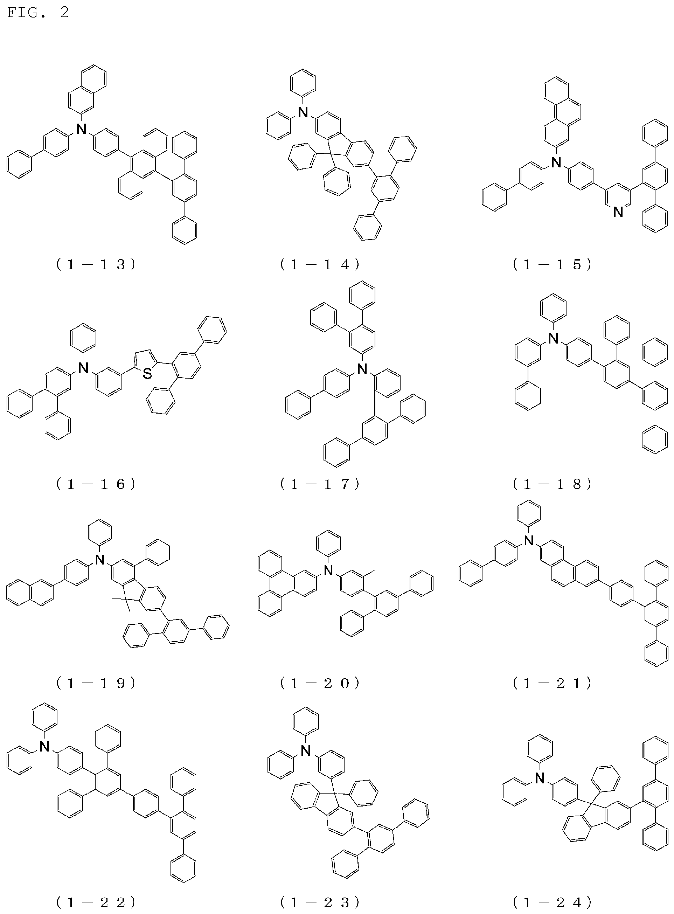

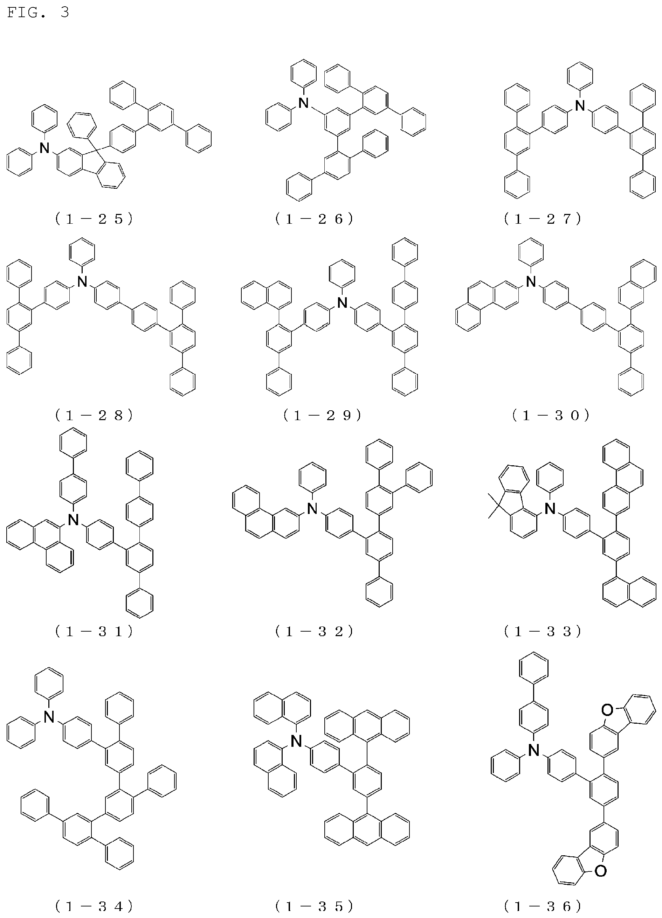

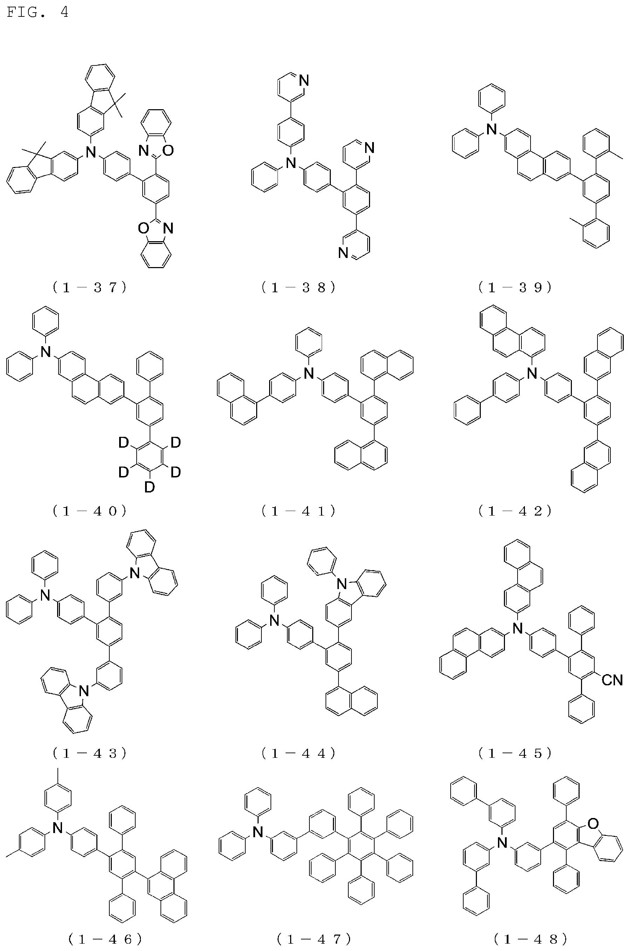

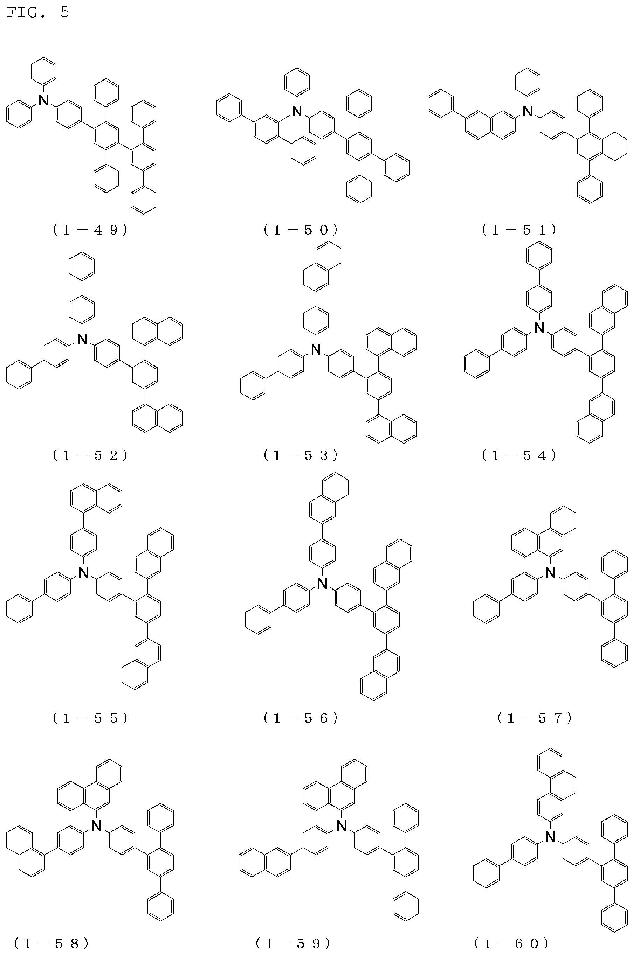

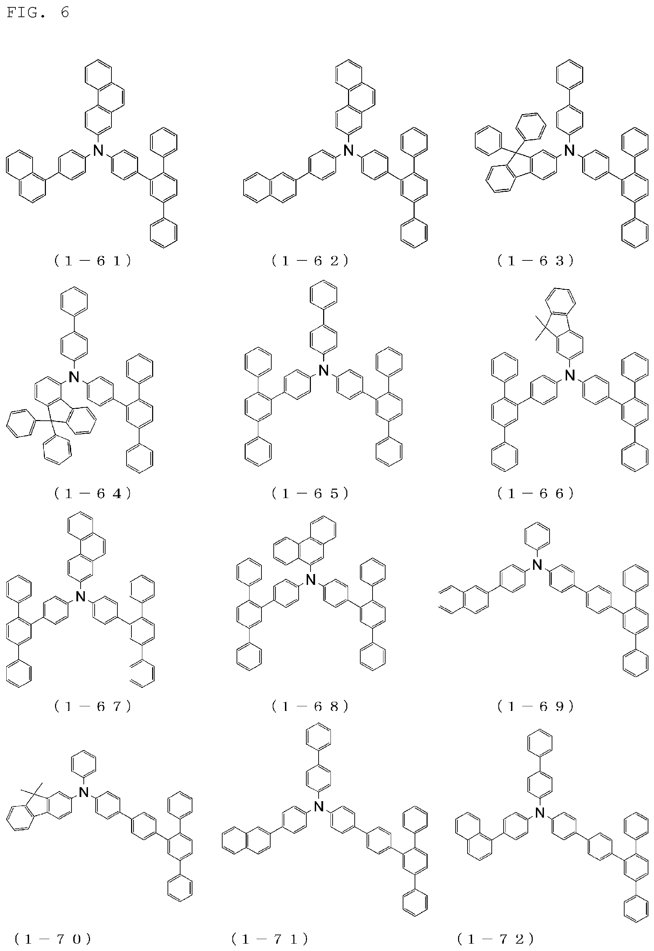

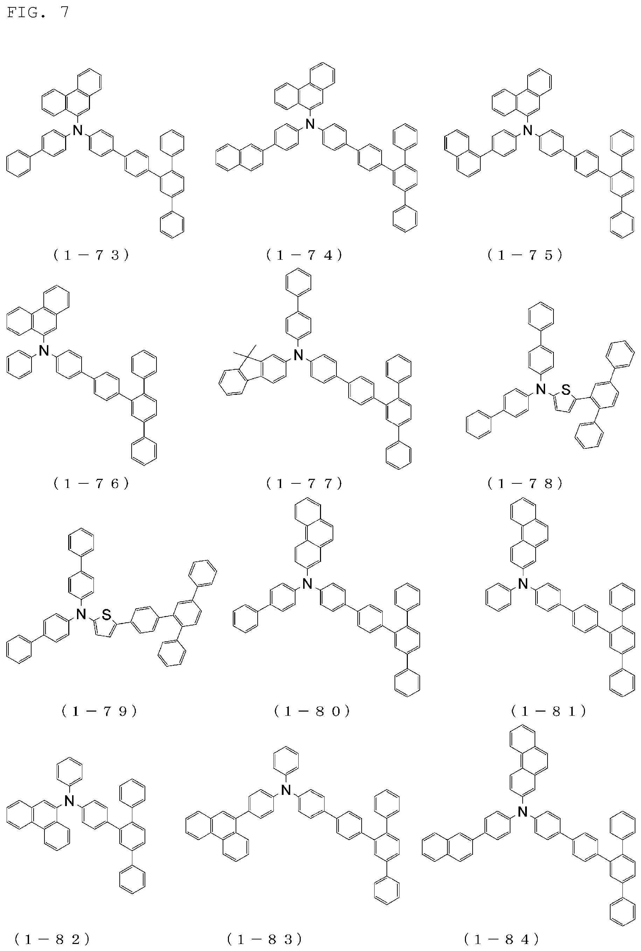

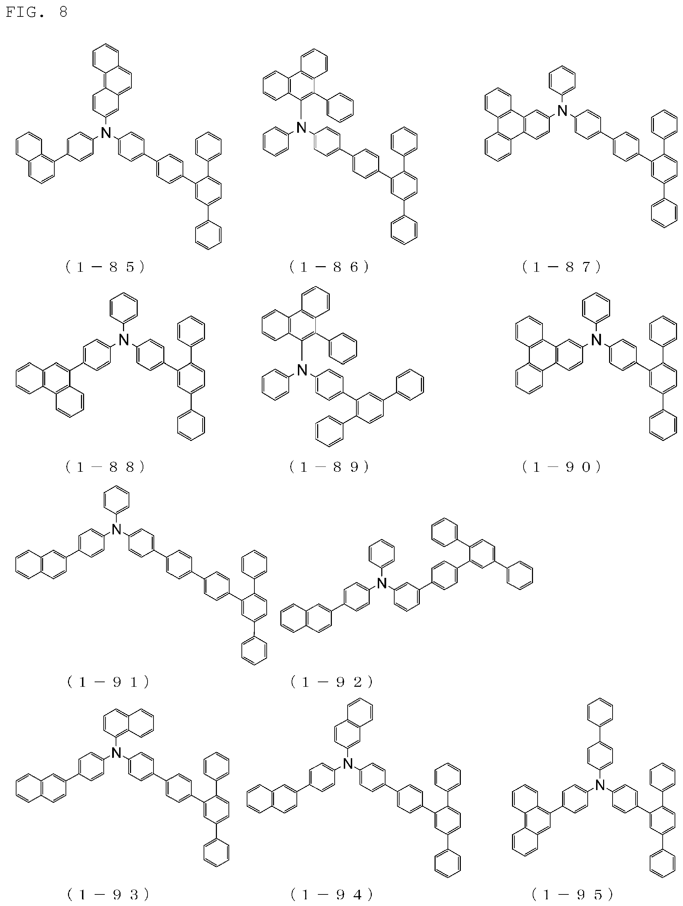

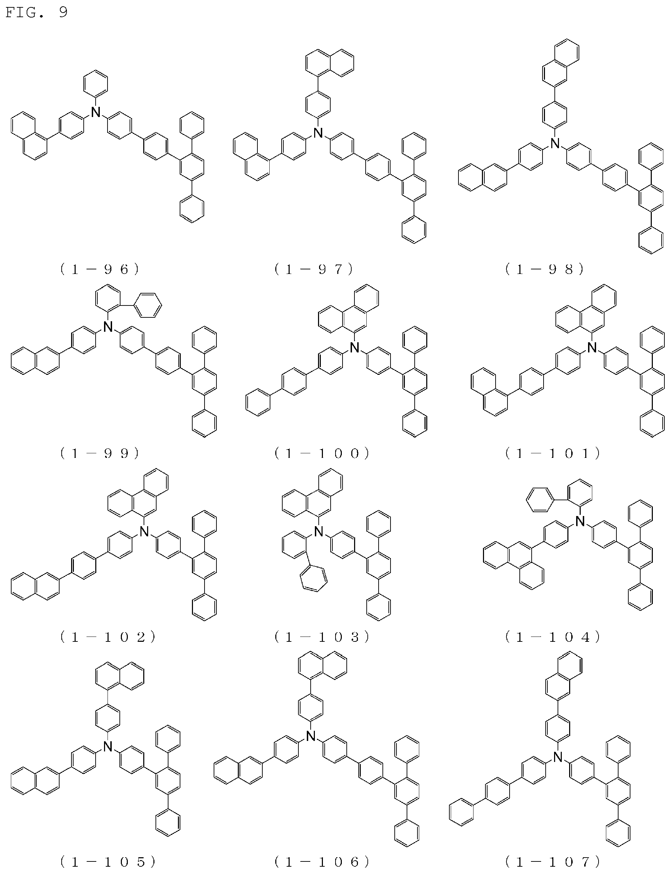

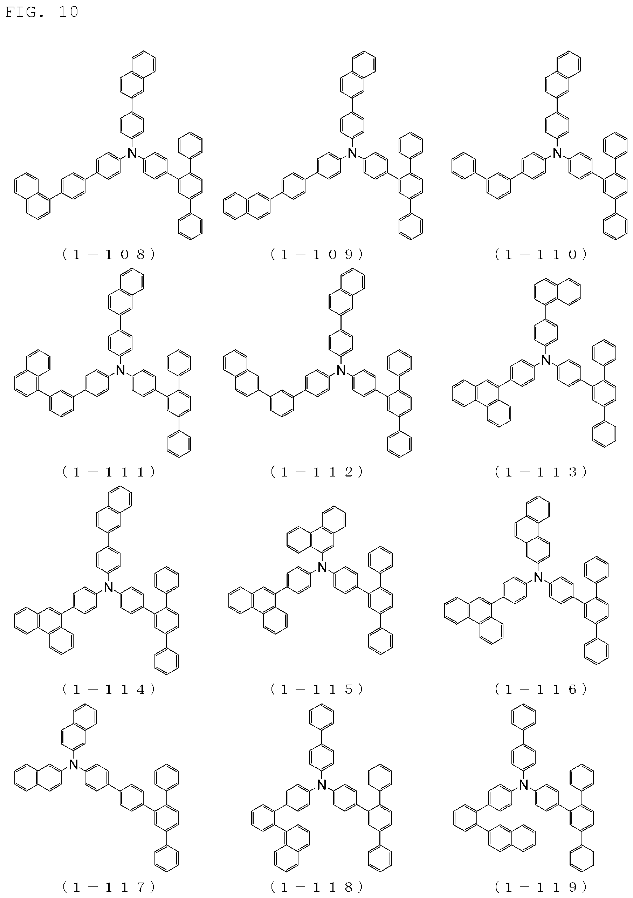

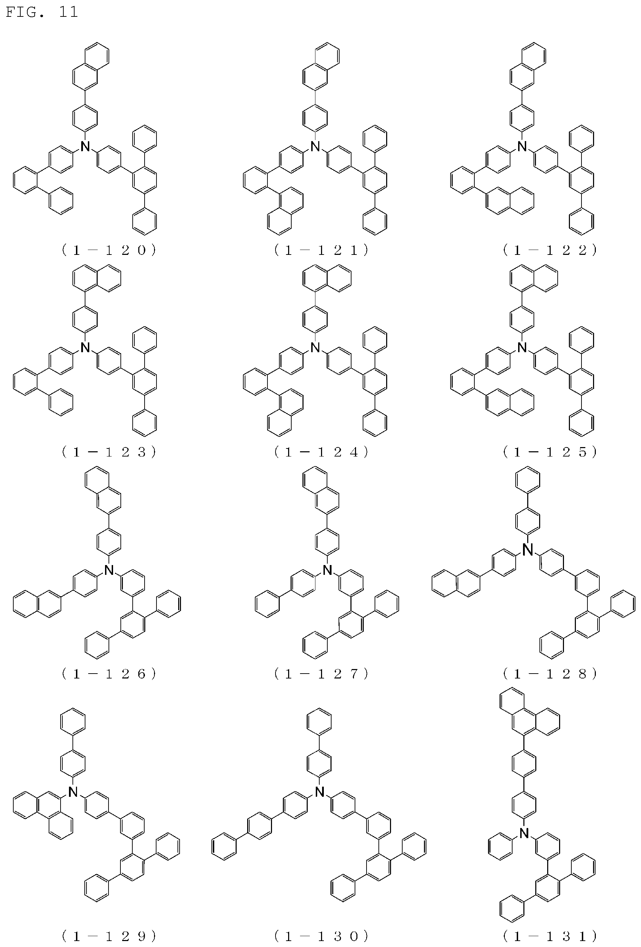

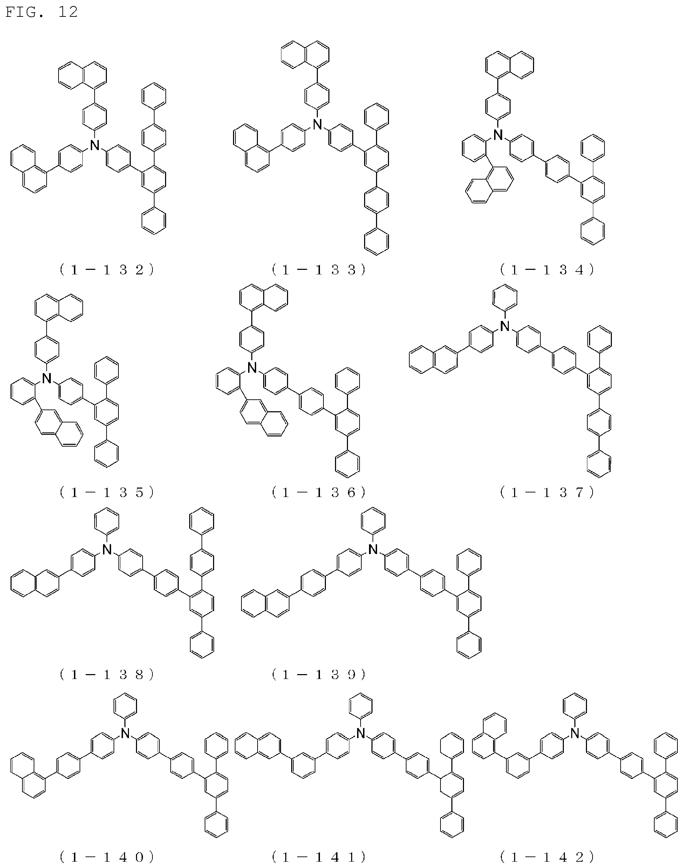

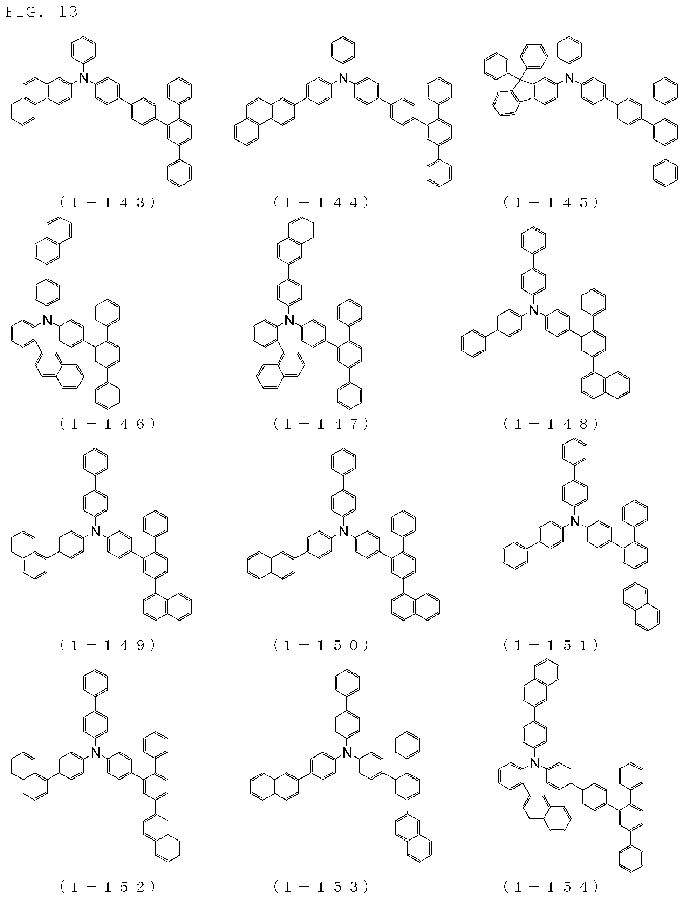

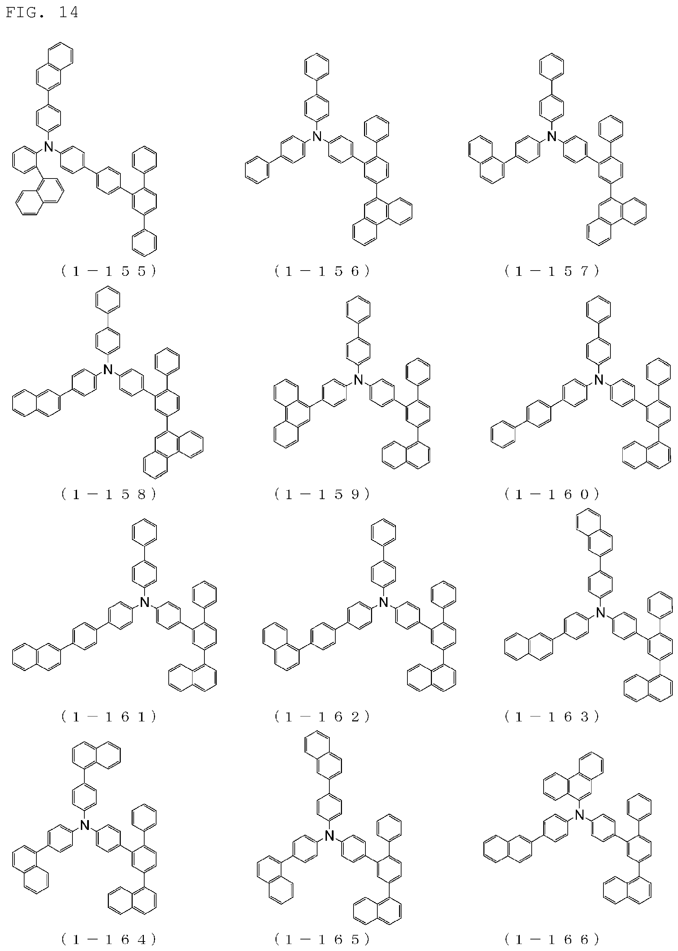

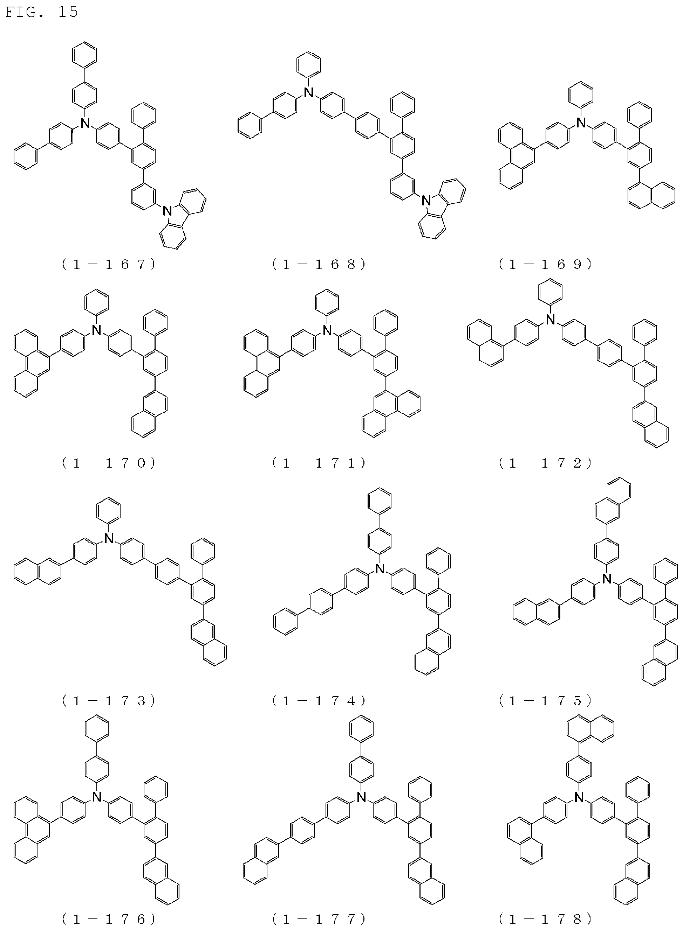

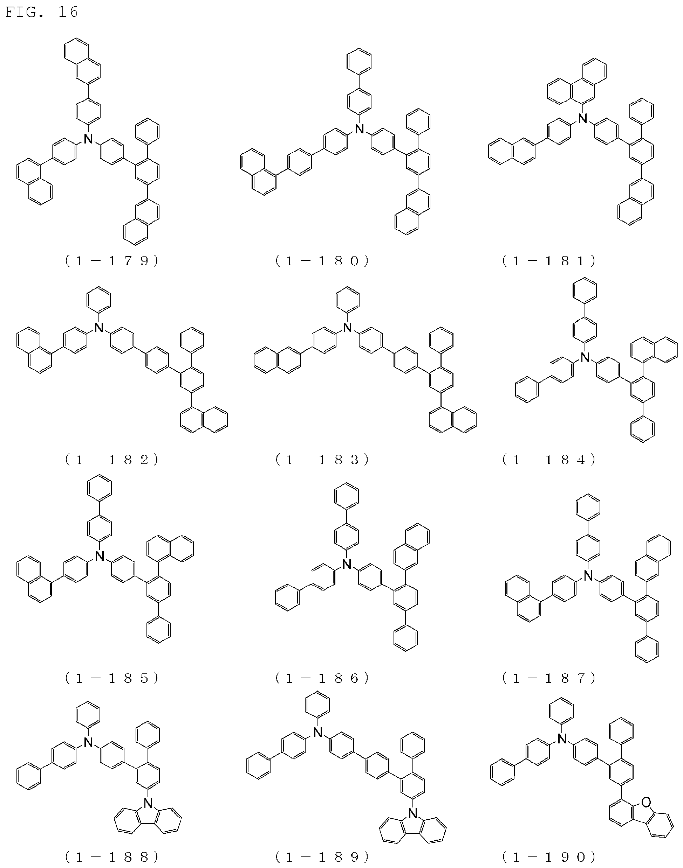

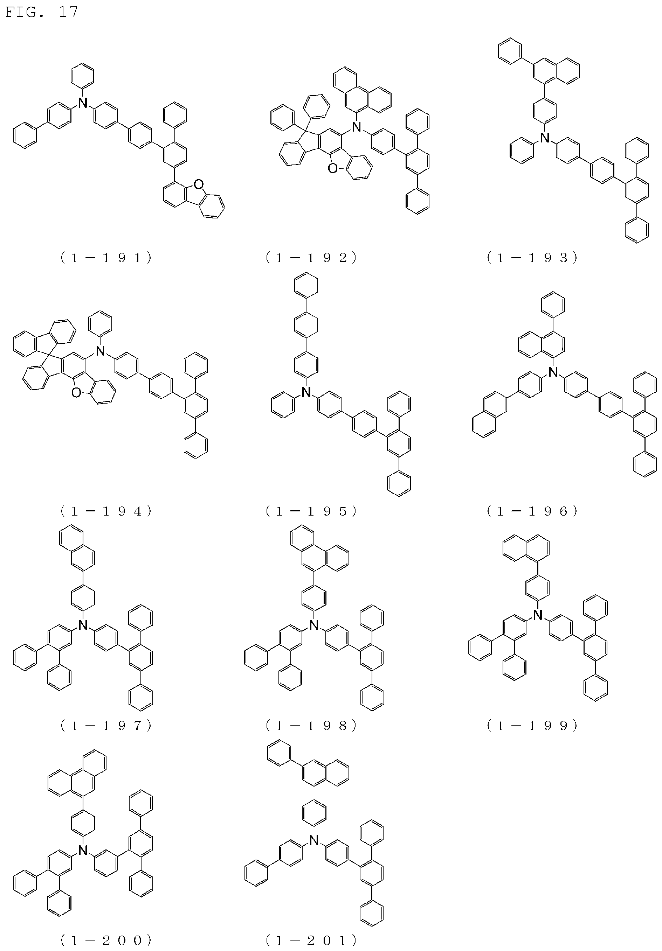

The specific examples of preferred compounds among the triarylamine compounds represented by the general formula (1) preferably used in the organic EL device of the present invention are shown in Figs. 1 to 17. The present invention, however, is not restricted to these compounds.

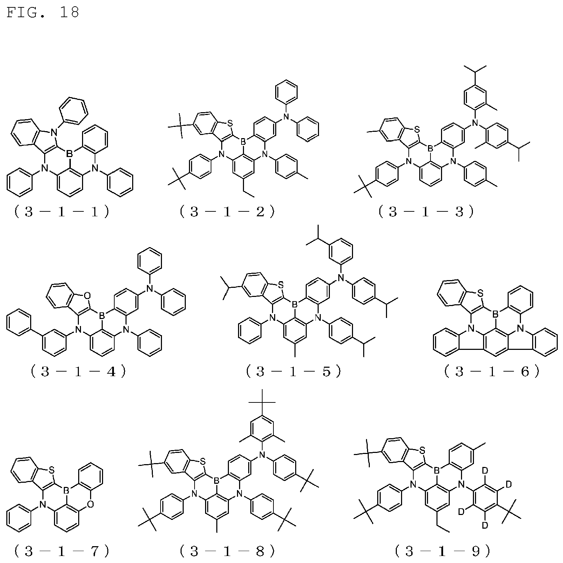

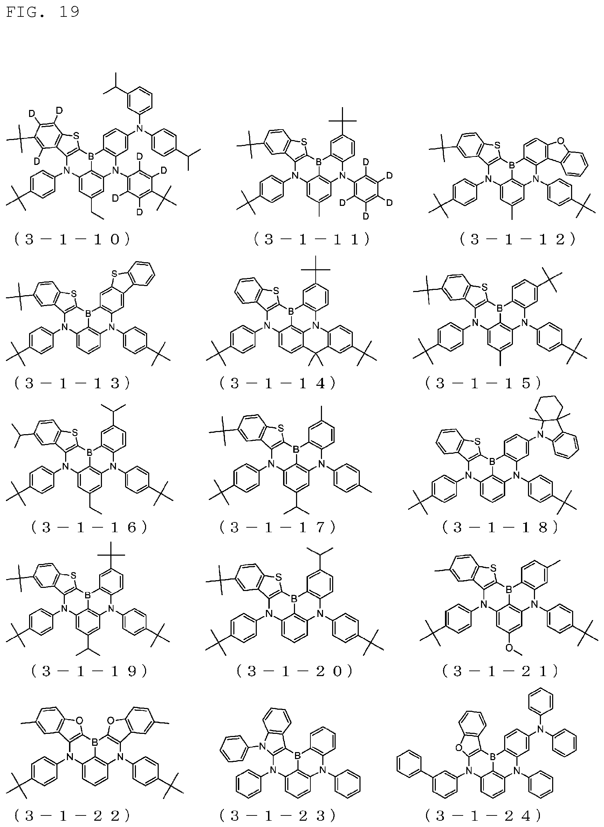

The specific examples of preferred compounds among the compounds represented by the general formula (3-1) or the general formula (3-2) preferably used in the organic EL device of the present invention are shown in Figs. 18 to 19 or 20, except compound 3-1-22 which is not in accordance with the present invention. The present invention, however, is not restricted to these compounds.

The triarylamine compounds of the general formula (1) were purified by methods such as column chromatography, adsorption using, for example, a silica gel, activated carbon, or activated clay, recrystallization or crystallization using a solvent, and a sublimation purification method. The compounds were identified by an NMR analysis. A glass transition point (Tg), and a work function were measured as material property values. The glass transition point (Tg) can be used as an index of stability in a thin-film state, and the work function can be used as an index of hole transportability and electron blocking performance. Other compounds used for the organic EL device of the present invention were purified by methods such as column chromatography, adsorption using, for example, a silica gel, activated carbon, or activated clay, and recrystallization or crystallization using a solvent, and finally purified by a sublimation purification method.

The glass transition point (Tg) was measured by a highsensitive differential scanning calorimeter (DSC3100SA produced by Bruker AXS) using powder.

For the measurement of the work function, a 100 nmthickness thin film was fabricated on an ITO substrate, and an ionization potential measuring device (PYS-202 produced by Sumitomo Heavy Industries, Ltd.) was used.

The organic EL device of the present invention may have a structure including an anode, a hole transport layer, a light emitting layer, an electron transport layer, and a cathode successively formed on a substrate, optionally with a hole injection layer between the anode and hole transport layer, a hole blocking layer between the light emitting layer and the electron transport layer, and an electron injection layer between the electron transport layer and the cathode. Some of the organic layers in the multilayer structure may be omitted, or may serve more than one function. For example, a single organic layer may serve as the hole injection layer and the hole transport layer, or as the electron injection layer and the electron transport layer, and so on. Further, any of the layers may be configured to laminate two or more organic layers having the same function, and the hole transport layer may have a two-layer laminated structure, the light emitting layer may have a two-layer laminated structure, the electron transport layer may have a two-layer laminated structure, and so on. The organic EL device of the present invention is preferably configured such that the hole transport layer has a two-layer laminated structure of a first hole transport layer and a second hole transport layer. In this case, the second hole transport layer is preferably adjacent to a light emitting layer, and it can function as an electron blocking layer.

Electrode materials with high work functions such as ITO and gold are used as the anode of the organic EL device of the present invention. The hole injection layer of the organic EL device of the present invention is preferably made of arylamine compounds having a structure in which two triphenylamine structures are joined within a molecule via a single bond or a divalent group that does not contain a heteroatom. Furthermore, material such as starburst-type triphenylamine derivatives and various triphenylamine tetramers; porphyrin compounds as represented by copper phthalocyanine; accepting heterocyclic compounds such as hexacyano azatriphenylene; and coating-type polymer materials can be used. These materials may be formed into a thin film by a vapor deposition method or other known methods such as a spin coating method and an inkjet method.

The triarylamine compounds of the general formula (1) are used as the hole transport layer of the organic EL device of the present invention. Examples of a hole transporting material that can be mixed or can be used at the same time with the triarylamine compounds of the general formula (1) can be the organic amine compounds such as various triphenylamine derivatives such as arylamine compounds having a structure in which four triphenylamine structures are joined within a molecule via a single bond or a divalent group that does not contain a heteroatom; and arylamine compounds having a structure in which two triphenylamine structures are joined within a molecule via a single bond or a divalent group that does not contain a heteroatom, in addition to benzidine derivatives such as N,N'-diphenyl-N,N'-di(m-tolyl)benzidine (TPD), N,N'-diphenyl-N,N'-di(α-naphthyl)benzidine (NPD), and N,N,N',N'-tetrabiphenylylbenzidine; and 1,1-bis[4-(di-4-tolylamino)phenyl]cyclohexane (TAPC). These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin film by a vapor deposition method or other known methods such as a spin coating method and an inkjet method.

The material used for the hole injection layer or the hole transport layer may be obtained by p-doping materials such as trisbromophenylamine hexachloroantimony, and radialene derivatives (refer to Patent Document 6, for example) into a material commonly used for these layers, or may be, for example, polymer compounds each having, as a part of the compound structure, a structure of a benzidine derivative such as TPD.

In the case where the hole transport layer of the organic EL device of the present invention has a two-layer structure of a first hole transport layer and a second hole transport layer, the triarylamine compounds of the general formula (1) are used as the second hole transport layer located on the light emitting layer side. Examples of a hole transporting material that can be mixed or can be used at the same time with the triarylamine compounds of the general formula (1) can be compounds having an electron blocking effect, including, for example, carbazole derivatives such as 4,4',4"tri(N-carbazolyl)triphenylamine (TCTA), 9,9-bis[4-(carbazol-9-yl)phenyl]fluorene, 1,3-bis(carbazol-9-yl)benzene (mCP), and 2,2-bis[4-(carbazol-9-yl)phenyl]adamantane (Ad-Cz); and compounds having a triphenylsilyl group and a triarylamine structure, as represented by 9-[4-(carbazol-9-yl)phenyl]-9-[4-(triphenylsilyl)phenyl]-9H-fluorene.

These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin film by using a vapor deposition method or other known methods such as a spin coating method and an inkjet method.

Material used for the blue light emitting layer of the organic EL device of the present invention includes a blue light emitting dopant which is a compound represented by the general formula (3-1) or the general formula (3-2). Furthermore, in addition to the quinolinol derivative metal complexes such as Alq₃; various metal complexes; anthracene derivatives; bis(styryl)benzene derivatives; pyrene derivatives; oxazole derivatives; and polyparaphenylene vinylene derivatives can be used. Further, the light emitting layer may be made of a host material and a dopant material. In that case, as the host material, an anthracene derivative having an anthracene skeleton in the molecule is preferably used. Furthermore, various metal complexes; bis(styryl)benzene derivatives; pyrene derivatives; oxazole derivatives; polyparaphenylene vinylene derivatives; heterocyclic compound having an indole ring as a partial structure of the fused ring; heterocyclic compound having a carbazole ring as a partial structure of fused ring; carbazole derivatives; thiazole derivatives; benzimidazole derivatives; and polydialkylfluorene derivatives can be used. Further, as the dopant material, a compound represented by the general formula (3-1), or the general formula (3-2) is used. Furthermore, a pyrene derivative having a pyrene skeleton in the molecule; heterocyclic compound having an indole ring as a partial structure of the fused ring; heterocyclic compound having a carbazole ring as a partial structure of fused ring; carbazole derivative; thiazole derivative; benzimidazole derivative; polydialkylfluorene derivative; quinacridone, coumarin, rubrene, perylene, derivatives thereof; benzopyran derivative; indenophenanthrene derivatives; rhodamine derivatives; and aminostyryl derivatives can be used. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer.

Further, a phosphorescent material can be used as a light-emitting material. Phosphorescent materials as metal complexes of metals such as iridium and platinum may be used. Examples of the phosphorescent materials include green phosphorescent materials such as Ir(ppy)₃, blue phosphorescent materials such as FIrpic and FIr6, and red phosphorescent materials such as Btp₂Ir(acac). Here, an anthracene derivative having an anthracene skeleton in the molecule is preferably used for the host material. Furthermore, carbazole derivatives such as 4,4'-di(N-carbazolyl)biphenyl (CBP), TCTA, and mCP may be used as the hole injecting and transporting host material. Compounds such as p-bis(triphenylsilyl)benzene (UGH2) and 2,2',2"-(1,3,5-phenylene)-tris(1-phenyl-1H-benzimidazole) (TPBI) may be used as the electron transporting host material. In this way, a highperformance organic EL device can be produced.

In order to avoid concentration quenching, the doping of the host material with the phosphorescent light-emitting material should preferably be made by co-evaporation in a range of 1 to 30 weight percent with respect to the whole light emitting layer.

Further, Examples of the light-emitting material may be delayed fluorescent-emitting material such as a CDCB derivative of PIC-TRZ, CC2TA, PXZ-TRZ, 4CzIPN or the like (refer to Non-Patent Document 3, for example).

These materials may be formed into a thin film by using a vapor deposition method or other known methods such as a spin coating method and an inkjet method.

The hole blocking layer of the organic EL device of the present invention may be formed by using hole blocking compounds such as various rare earth complexes, triazole derivatives, triazine derivatives, and oxadiazole derivatives, in addition to the metal complexes of phenanthroline derivatives such as bathocuproin (BCP), and the metal complexes of quinolinol derivatives such as aluminum(III) bis(2-methyl-8-quinolinate)-4-phenylphenolate (BAlq). These materials may also serve as the material of the electron transport layer. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin film by using a vapor deposition method or other known methods such as a spin coating method and an inkjet method.

The electron transport layer of the organic EL device of the present invention may be formed by using metal complexes of quinolinol derivatives such as Alq₃ and BAlq, various metal complexes, triazole derivatives, triazine derivatives, oxadiazole derivatives, pyridine derivatives, pyrimidine derivatives, benzimidazole derivatives, thiadiazole derivatives, anthracene derivatives, carbodiimide derivatives, quinoxaline derivatives, pyridoindole derivatives, phenanthroline derivatives, and silole derivatives. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin film by using a vapor deposition method or other known methods such as a spin coating method and an inkjet method.

Examples of material used for the electron injection layer of the organic EL device of the present invention can be alkali metal salts such as lithium fluoride and cesium fluoride; alkaline earth metal salts such as magnesium fluoride; metal complexes of quinolinol derivatives such as lithium quinolinol; metal oxides such as aluminum oxide; and metals such as ytterbium (Yb), samarium (Sm), calcium (Ca), strontium (Sr), cesium (Cs). However, the electron injection layer may be omitted in the preferred selection of the electron transport layer and the cathode.

Further, in the electron injection layer or the electron transport layer, a material obtained by further N-doping a material which is commonly used for the layer with a metal such as cesium, or the like can be used.

The cathode of the organic EL device of the present invention may be made of an electrode material with a low work function such as aluminum, or an alloy of an electrode material with an even lower work function such as a magnesium-silver alloy, a magnesium-indium alloy, or an aluminum-magnesium alloy.

Material used for the capping layer of the organic EL device of the present invention can be preferably arylamine compounds having a structure in which 2 to 6 triphenylamine structures are joined within a molecule via a single bond or a divalent group that does not contain a heteroatom; amine compound having a benzoazole ring structure; or amine compound having an aromatic heterocyclic group in the molecule. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin film by using a vapor deposition method or other known methods such as a spin coating method and an inkjet method.

The following describes an embodiment of the present invention in more detail based on Examples. The present invention, however, is not restricted to the following Examples.

### Example 1

<Synthesis of bis(4-naphthalen-2-yl-phenyl)-(2',5'-diphenyl-biphenyl-4-yl)amine (Compound 1-4)> bis(4-naphthalen-2-yl-phenyl) amine: 10.0 g, 4-bromo-2',5'-diphenyl-biphenyl: 11.0 g, palladium(II) acetate: 0.1 g, tri(tert-butyl)phosphine: 0.2 g, tert-butoxy sodium: 2.7 g were added into a reaction vessel, and the mixture was stirred at reflux in toluene solvent for 3 hours. After allowing to cool, the filtrate obtained by filtration was concentrated to obtain a crude product. The crude product was purified by crystallization with a mixed solvent of toluene / acetone, whereby a white powder of bis(4-naphthalen-2-yl-phenyl)-(2',5'-diphenylbiphenyl-4-yl)amine (Compound 1-4): 9.0 g (yield: 52.3%) was obtained.

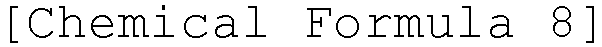

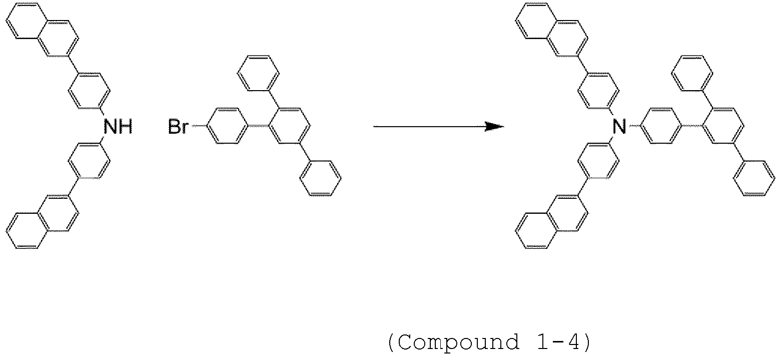

¹H-NMR (CDCl₃) of the obtained white powder detected 39 hydrogen signals, as follows, and the structure identified.
is (ppm) = 8.06 (2H), 7.92 (6H), 7.78 (4H), 7.73 (1H), 7.68 (5H), 7.53 (7H), 7.42 (1H), 7.39-7.23 (9H), 7.14 (4H).

### Example 2

### <Synthesis of (2',5'-diphenyl-biphenyl-4-yl)-(4-naphthalen-1-yl-phenyl)-phenanthrene-9-yl-amine (Compound 1-58)>

(2',5'-diphenyl-biphenyl-4-yl)-(4-naphthalen-1-yl-phenyl)amine: 8.5 g, 9-bromo-phenanthrene: 4.8 g, palladium(II) acetate: 0.1 g, tri(tert-butyl)phosphine: 0.3 g, tert-butoxy sodium: 2.3 g were added into a reaction vessel, and the mixture was stirred at reflux in toluene solvent for 3 hours. After allowing to cool, the filtrate obtained by filtration was concentrated to obtain a crude product. The crude product was purified by crystallization with a mixed solvent of toluene / acetone, whereby a white powder of (2',5'-diphenyl-biphenyl-4-yl)-(4-naphthalen-1-yl-phenyl)-phenanthrene-9-yl-amine (Compound 1-58): 8.3 g (yield: 73.1%) was obtained.

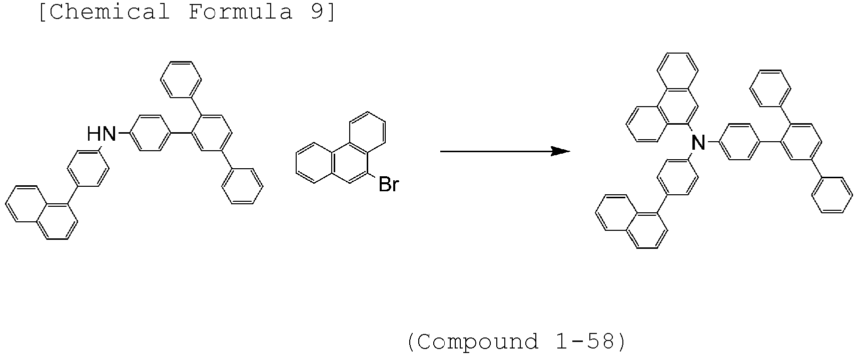

¹H-NMR (CDCl₃) of the obtained white powder detected 37 hydrogen signals, as follows, and the structure identified.
δ (ppm) = 8.79 (1H), 8.75 (1H), 8.14 (1H), 8.03 (1H), 7.92 (1H), 7.85 (2H), 7.72 (6H), 7.65 (2H), 7.60 (1H), 7.50 (7H), 7.42 (1H), 7.36 (3H), 7.27-7.18 (6H), 7.09(4H).

### Example 3

### <Synthesis of (2',5'-diphenyl-biphenyl-4-yl)-(4-naphthalen-2-yl-phenyl)-phenanthrene-9-yl-amine (Compound 1-59)>

(2',5'-diphenyl-biphenyl-4-yl)-(4-naphthalen-2-yl-phenyl)amine: 8.0 g, 9-bromo-phenanthrene: 4.5 g, palladium(II) acetate: 0.1 g, tri(tert-butyl)phosphine: 0.2 g, tert-butoxy sodium: 2.2 g were added into a reaction vessel, and the mixture was stirred at reflux in toluene solvent for 3 hours. After allowing to cool, the filtrate obtained by filtration was concentrated to obtain a crude product. The crude product was purified by crystallization with a mixed solvent of toluene / acetone, whereby a pale yellow powder of (2',5'-diphenyl-biphenyl-4-yl)-(4-naphthalen-2-yl-phenyl)-phenanthrene-9-yl-amine (Compound 1-59): 6.6 g (yield: 61.7%) was obtained.

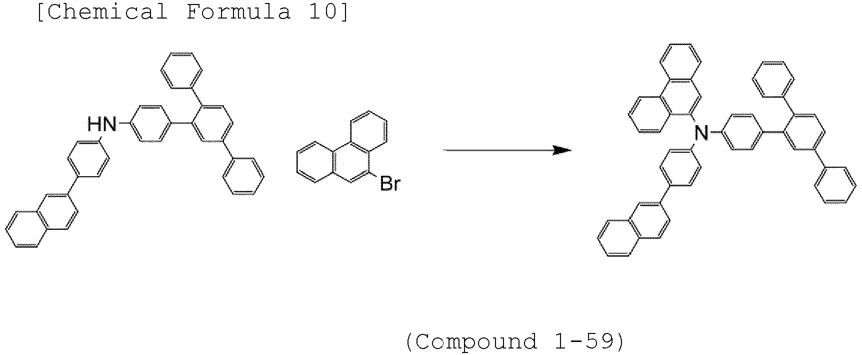

¹H-NMR (CDCl₃) of the obtained pale yellow powder detected 37 hydrogen signals, as follows, and the structure identified.
δ (ppm) = 8.79 (1H), 8.74 (1H), 8.09 (1H), 8.01 (1H), 7.86 (4H), 7.75 (1H), 7.71 (5H), 7.66 (2H), 7.60 (3H), 7.50 (5H), 7.39 (1H), 7.34-7.23 (6H), 7.20 (2H), 7.07 (4H).

### Example 4

### <Synthesis of (2'',5"-diphenyl-[1,1';4',1"]terphenyl-4-yl)-(4-naphthalen-2-yl-phenyl)-phenylamine (Compound 1-69)>

(4-naphthalen-2-yl-phenyl)-phenylamine: 6.0 g, 4-bromo-2'',5''-diphenyl-[1,1';4',1'']terphenyl: 10.3 g, palladium(II) acetate: 0.1 g, tri(tert-butyl)phosphine: 0.2 g, tert-butoxy sodium: 2.3 g were added into a reaction vessel, and the mixture was stirred at reflux in toluene solvent for overnight. After allowing to cool, the filtrate obtained by filtration was concentrated to obtain a crude product. The crude product was purified using column chromatography (support: silica gel, eluent: dichloromethane / n-heptane), whereby a white powder of (2",5"-diphenyl-[1,1';4',1'']terphenyl-4-yl)-(4-naphthalen-2-yl-phenyl)-phenylamine (Compound 1-69): 7.1 g (yield: 51.7%) was obtained.

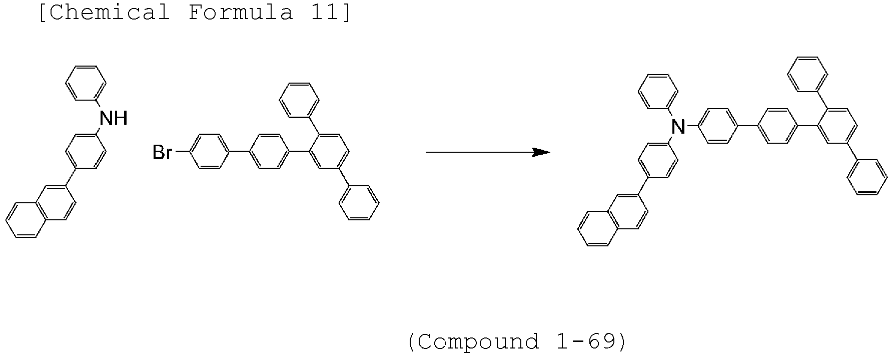

¹H-NMR (CDCl₃) of the obtained white powder detected 37 hydrogen signals, as follows, and the structure identified.
δ (ppm) = 8.04 (1H), 7.91 (3H), 7.73 (5H), 7.66 (2H), 7.56 (2H), 7.51 (7H), 7.42 (1H), 7.39-7.18 (15H), 7.10(1H).

### Example 5

### <Synthesis of (2",5"-diphenyl-[1,1';4',1"]terphenyl-4-yl)-(4-phenanthrene-9-yl-phenyl)-phenylamine (Compound 1-83)>

(4-phenanthrene-9-yl-phenyl)-phenylamine: 11.0 g, 4-bromo-2'',5"-diphenyl-[1,1';4',1"]terphenyl: 16.2 g, palladium(II) acetate: 0.1 g, tri(tert-butyl)phosphine: 0.3 g, tert-butoxy sodium: 3.7 g were added into a reaction vessel, and the mixture was stirred at reflux in toluene solvent for overnight. After allowing to cool, the filtrate obtained by filtration was concentrated to obtain a crude product. The crude product was purified using column chromatography (support: silica gel, eluent: dichloromethane / n-heptane), whereby a white powder of (2",5"-diphenyl-[1,1';4',1"]terphenyl-4-yl)-(4-phenanthrene-9-yl-phenyl)-phenylamine (Compound 1-83): 11.2 g (yield: 48.5%) was obtained.

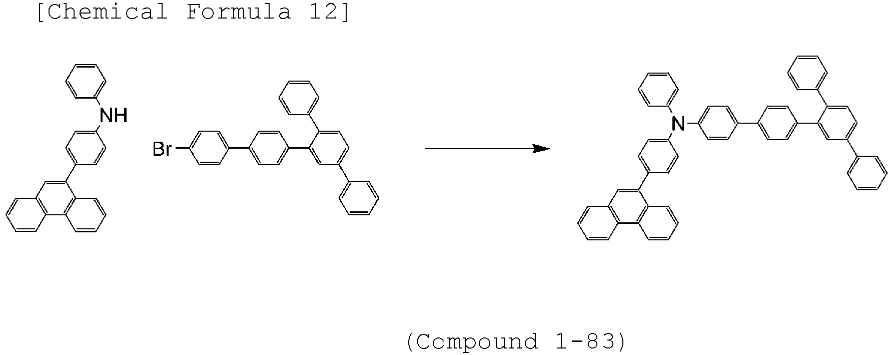

¹H-NMR (CDCl₃) of the obtained white powder detected 39 hydrogen signals, as follows, and the structure identified.
is (ppm) = 8.81 (1H), 8.75 (1H), 8.09 (1H), 7.93 (1H), 7.71 (7H), 7.65-7.44 (10H), 7.44-7.22 (17H), 7.11 (1H).

### Example 6

### <Synthesis of (2',5'-diphenyl-biphenyl-4-yl)-(4'-naphthalen-1-yl-biphenyl-4-yl)-(4-naphthalen-2-yl-phenyl)amine (Compound 1-108)>

4-bromophenyl-(2',5'-diphenyl-biphenyl-4-yl)-(4-naphthalen-2-yl-phenyl)amine: 11.0 g, 4-naphthalen-1-yl-phenylboronic acid: 4.8 g, tetrakis(triphenylphosphine)palladium (0): 0.4 g, potassium carbonate: 4.5 g were added into a reaction vessel, and the mixture was stirred at reflux in a mixed solvent of toluene / ethanol / water for overnight. After allowing to cool, a crude product precipitated by adding methanol was collected by filtration. The crude product was purified by crystallization with a mixed solvent of toluene / acetone, whereby a white powder of (2',5'-diphenyl-biphenyl-4-yl)-(4'-naphthalen-1-yl-biphenyl-4-yl)-(4-naphthalen-2-yl-phenyl)amine (Compound 1-108): 11.0 g (yield: 84.6%) was obtained.

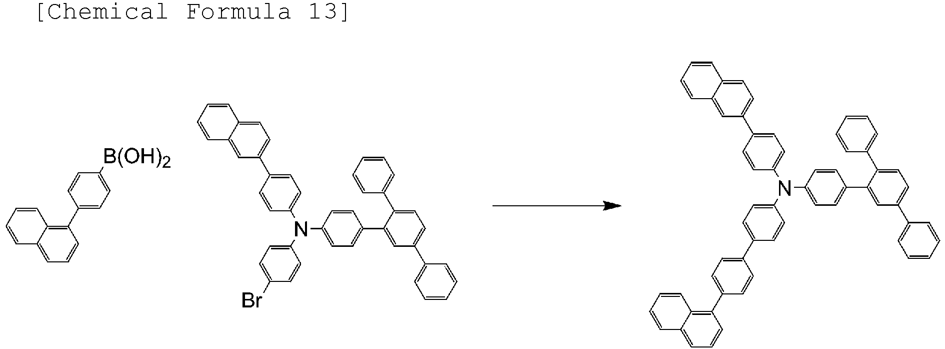

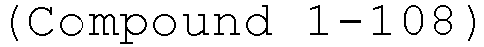

¹H-NMR (CDCl₃) of the obtained white powder detected 43 hydrogen signals, as follows, and the structure identified.
δ (ppm) = 8.04 (1H), 7.99 (1H), 7.92 (1H), 7.90 (1H), 7.87 (3H), 7.76 (1H), 7.74-7.69 (5H), 7.65 (3H), 7.61 (2H), 7.57 (2H), 7.54 (1H), 7.53-7.42 (8H), 7.38 (1H), 7.34-7.21 (9H), 7.11 (4H).

### Example 7

### <Synthesis of (2',5'-diphenyl-biphenyl-4-yl)-(3'-naphthalen-2-yl-biphenyl-4-yl)-(4-naphthalen-2-yl-phenyl)amine (Compound 1-112)>

4-bromophenyl-(2',5'-diphenyl-biphenyl-4-yl)-(4-naphthalen-2-yl-phenyl)amine: 12.0 g, 3-naphthalen-2-yl-phenylboronic acid: 5.3 g, tetrakis(triphenylphosphine)palladium (0): 0.4 g, potassium carbonate: 4.9 g were added into a reaction vessel, and the mixture was stirred at reflux in a mixed solvent of toluene / ethanol / water for overnight. After allowing to cool, a crude product precipitated by adding methanol was collected by filtration. The crude product was purified by crystallization with a mixed solvent of toluene / acetone, whereby a white powder of (2',5'-diphenyl-biphenyl-4-yl)-(3'-naphthalen-2-yl-biphenyl-4-yl)-(4-naphthalen-2-yl-phenyl)amine (Compound 1-112): 11.3 g (yield: 79.7%) was obtained.

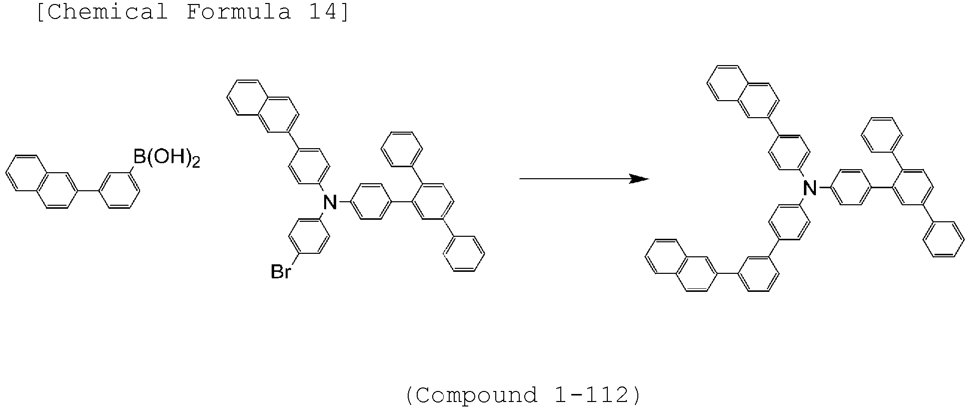

¹H-NMR (CDCl₃) of the obtained white powder detected 43 hydrogen signals, as follows, and the structure identified.
δ (ppm) = 8.11 (1H), 8.03 (1H), 7.97-7.93 (7H), 7.81 (1H), 7.78-7.57 (10H), 7.57-7.43 (8H), 7.37 (1H), 7.33-7.03 (14H).

### Example 8

### <Synthesis of (9,9-diphenyl-9H-fluorene-2-yl)-(2'',5''-diphenyl-[1,1';4',1'']terphenyl-4-yl)-phenylamine (Compound 1-145)>

(2",5"-diphenyl-[1,1';4',1"]terphenyl-4-yl)-phenylamine: 11.0 g, 2-bromo-9,9-diphenyl-9H-fluorene: 10.2 g, palladium(II) acetate: 0.1 g, tri(tert-butyl)phosphine: 0.2 g, tert-butoxy sodium: 2.7 g were added into a reaction vessel, and the mixture was stirred at reflux in toluene solvent for overnight. After allowing to cool, the filtrate obtained by filtration was concentrated to obtain a crude product. The crude product was purified using column chromatography (support: silica gel, eluent: dichloromethane / n-heptane), whereby a white powder of (9,9-diphenyl-9H-fluorene-2-yl)-(2'',5''-diphenyl-[1,1';4',1'']terphenyl-4-yl)-phenylamine (Compound 1-145): 15.0 g (yield: 81.9%) was obtained.

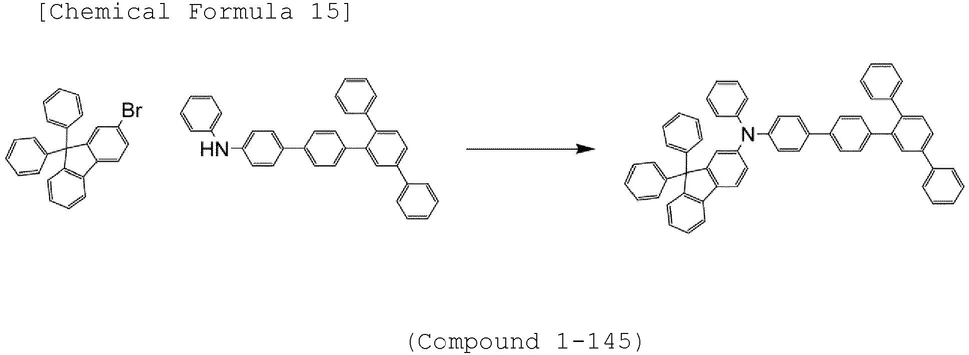

¹H-NMR (CDCl₃) of the obtained white powder detected 43 hydrogen signals, as follows, and the structure identified.
δ (ppm) = 7.71 (2H), 7.67 (3H), 7.60 (1H), 7.52 (1H), 7.50-7.40 (6H), 7.40-7.30 (3H), 7.27-7.13 (21H), 7.08 (4H), 7.04 (1H), 7.00 (1H).

### Example 9

### <Synthesis of biphenyl-4-yl-(5'-naphthalene-2-yl-[1,1';2',1"]terphenyl-4-yl)-([1,1';4',1"]terphenyl-4-yl)amine (Compound 1-174)>

biphenyl-4-yl-(5'-naphthalene-2-yl-[1,1';2',1'']terphenyl-4-yl)amine: 10.0 g, 4-bromo-[1,1';4',1'']terphenyl: 6.5 g, palladium(II) acetate: 0.1 g, tri(tert-butyl)phosphine: 0.2 g, tert-butoxy sodium: 2.2 g were added into a reaction vessel, and the mixture was stirred at reflux in toluene solvent for overnight. After allowing to cool, the filtrate obtained by filtration was concentrated to obtain a crude product. The crude product was purified by crystallization with a mixed solvent of toluene / acetone, whereby a white powder of biphenyl-4-yl-(5'-naphthalene-2-yl-[1,1';2',1"]terphenyl-4-yl)-([1,1';4',1'']terphenyl-4-yl)amine (Compound 1-174): 10.4 g (yield: 72.4%) was obtained.

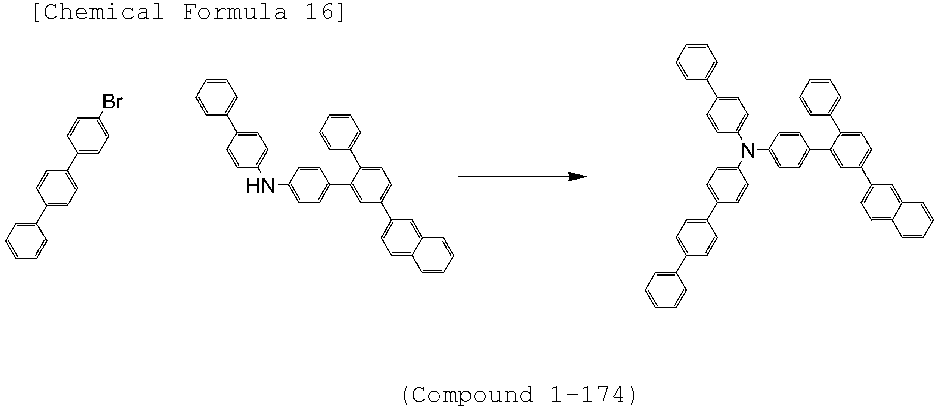

¹H-NMR (CDCl₃) of the obtained white powder detected 41 hydrogen signals, as follows, and the structure identified.
δ (ppm) = 8.15 (1H), 7.95 (1H), 7.92 (1H), 7.88 (1H), 7.85 (2H), 7.78 (1H), 7.67 (4H), 7.64 (2H), 7.60 (1H), 7.57 (3H), 7.55-7.48 (5H), 7.45 (2H), 7.43 (2H), 7.36 (1H), 7.34-7.26 (6H), 7.20 (4H), 7.15 (2H), 7.07 (2H).

### Example 10

### <Synthesis of compound (3-1-11)>

1-bromobenzene (D-substituted): 45.0 g, 4-tert-butylaniline: 58.0 g, palladium (II) acetate: 1.0 g, tert-butoxy sodium: 30.0 g, bis(diphenylphosphino)-1,1'-binaphthyl: 2.0 g, toluene: 450 mL were added into a reaction vessel, and the mixture was refluxed and stirred for 24 hours. After allowing to cool, the mixture was concentrated and purified using column chromatography, whereby a powder of the following compound (3-1-11a): 49.9 g (yield: 78%) was obtained.

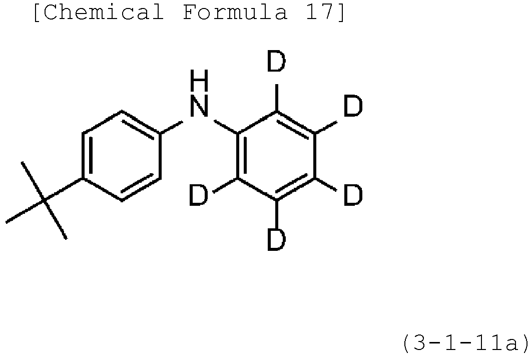

The above compound (3-1-11a): 20.0 g, the following compound (3-1-11b): 18.4 g, palladium (II) acetate: 0.5 g, tert-butoxy sodium: 18.9 g, tri(tert-butyl)phosphine: 0.8 g, toluene: 200 mL were added into a reaction vessel, and the mixture was refluxed and stirred for 24 hours. After allowing to cool, the mixture was concentrated and purified using column chromatography, whereby a powder of the following compound (3-1-11c): 21.5 g (yield: 84%) was obtained.

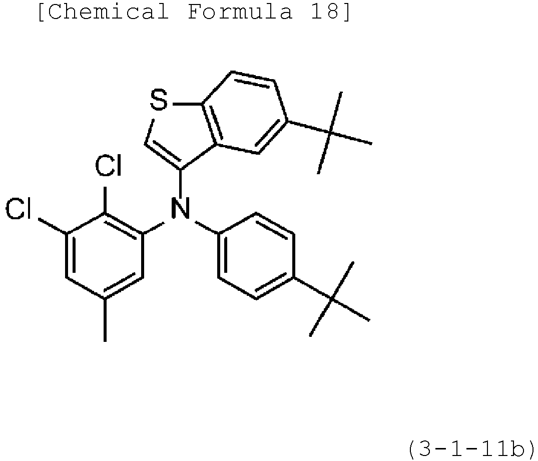

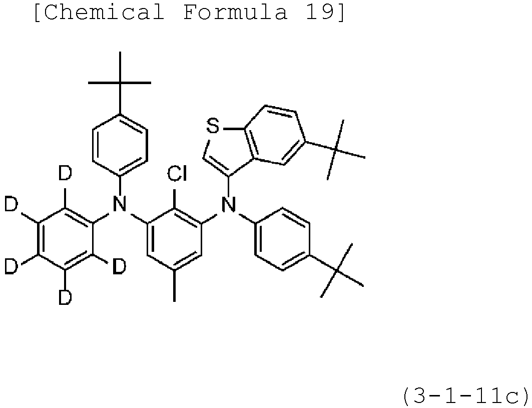

The above compound (3-1-11c): 12.0 g, and tertbutylbenzene: 120mL were added into a reaction vessel. Then, 42.5 mL of n-butyllithium was added thereto dropwise at -78 °C, and the mixture was aerated with nitrogen gas stirring at 60 °C for 3 hours. Then, 11.3 g of boron tribromid was added thereto dropwise at -78 °C, and the mixture was stirred at room temperature for 1 hour. Then,5.9 g of N, N-diisopropylethylamine was added thereto dropwise at 0 °C, and the mixture was stirred at 120 °C for 2 hours. After allowing to cool, an aqueous sodium acetate solution was added thereto, and the mixture is stirred. An organic layer was separated and extracted with ethyl acetate, and the organic layer was concentrated. The mixture was purified using column chromatography, whereby a powder of the following compound (3-1-11): 1.7 g (yield: 11%) was obtained.

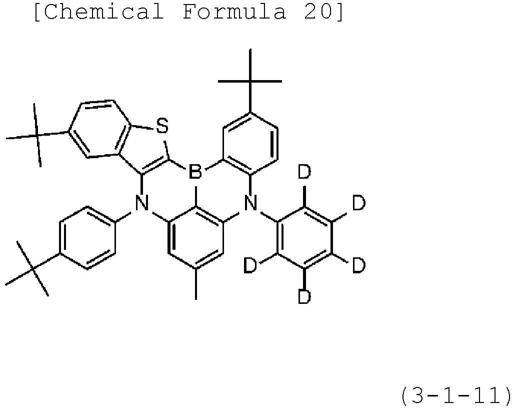

### Example 11

The glass transition points (Tg) of the triarylamine compounds of the general formula (1) were determined using a high-sensitive differential scanning calorimeter (DSC3100SA produced by Bruker AXS). The measurement results are shown below.

| | Glass transition point (Tg) |
|---|---|
| Compound of Example 1 | 107.1°C |
| Compound of Example 2 | 131.2°C |
| Compound of Example 3 | 129.7°C |
| Compound of Example 4 | 110.0°C |
| Compound of Example 5 | 127.9°C |
| Compound of Example 6 | 121.4°C |
| Compound of Example 7 | 109.5°C |
| Compound of Example 8 | 136.2°C |
| Compound of Example 9 | 116.1°C |

The triarylamine compounds of the general formula (1) have glass transition points (Tg) of 100 °C or higher, demonstrating that the compounds have a stable thin-film state.

### Example 12

A 100 nm-thick vapor-deposited film was fabricated on an ITO substrate using the triarylamine compounds of the general formula (1), and a work function was measured using an ionization potential measuring device (PYS-202 produced by Sumitomo Heavy Industries, Ltd.). The measurement results are shown below.

| | Work function |
|---|---|
| Compound of Example 1 | 5.67eV |
| Compound of Example 2 | 5.72eV |
| Compound of Example 3 | 5.75eV |
| Compound of Example 4 | 5.72eV |
| Compound of Example 5 | 5.76eV |
| Compound of Example 6 | 5.69eV |
| Compound of Example 7 | 5.69eV |
| Compound of Example 8 | 5.68eV |
| Compound of Example 9 | 5.69eV |

As the results show, the triarylamine compounds of the general formula (1) have desirable energy levels compared to the work function 5.4 eV of common hole transport materials such as NPD and TPD, and thus possess desirable hole transportability and an excellent electron blocking ability.

### Example 13

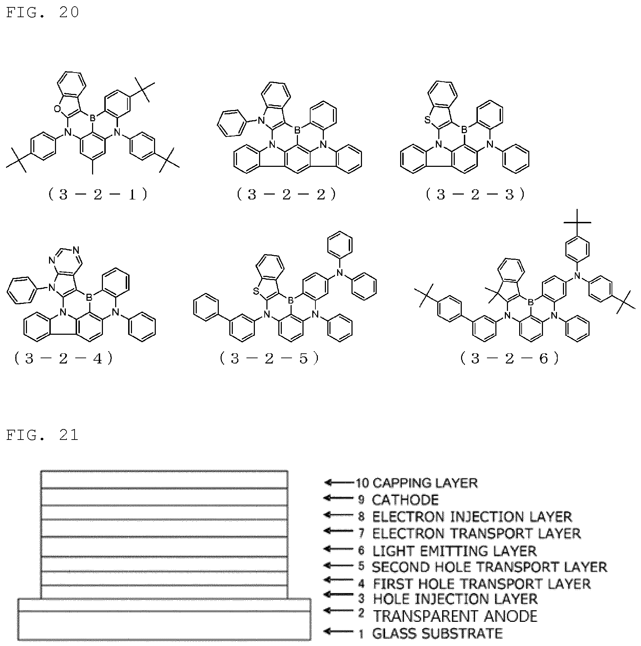

The organic EL device, as shown in FIG. 21, was fabricated by vapor-depositing a hole injection layer 3, a first hole transport layer 4, a second hole transport layer 5, a light emitting layer 6, an electron transport layer 7, an electron injection layer 8, a cathode 9 and capping layer 10 in this order on a glass substrate 1 on which an ITO electrode was formed as a transparent anode 2 beforehand.

Specifically, as the transparent anode 2, an ITO film with a thickness of 50 mm, a silver alloy reflective film with a thickness of 100 nm, and an ITO film with a thickness of 5 nm were deposited on the glass substrate 1 in this order. After ultrasonic cleaning in isopropyl alcohol for 20 minutes, the film was dried on a hot plate heated to 250 °C for 10 minutes. After UV ozone treatment for 15 minutes, the glass substrate with ITO was installed in a vacuum vapor deposition apparatus, and the pressure was reduced to 0.001 Pa or lower. Subsequently, as the hole injection layer 3 covering the transparent anode 2, an electron acceptor (Acceptor-1) of the structural formula below and compound (HTM-1) of the structural formula below were formed in a film thickness of 10 nm by dual vapor deposition at a vapor deposition rate ratio of Acceptor-1:compound (HTM-1) = 3:97. The first hole transport layer 4 was formed on the hole injection layer 3 by forming the compounds (HTM-1) of the structural formula below in a film thickness of 140 nm. The second hole transport layer 5 was formed on the first hole transport layer 4 by forming the compound (1-4) of Example 1 in a film thickness of 5 nm. Then, the light emitting layer 6 was formed on the second hole transport layer 5 in a film thickness of 20 nm by dual vapor deposition of the compound (3-1-11) of Example 10 and compound (EMH-1) of the structural formula below at a vapor deposition rate ratio of the compound (3-1-11):compound (EMH-1) = 5:95. The electron transport layer 7 was formed on the light emitting layer 6 in a film thickness of 30 nm by dual vapor deposition of the compound (ETM-1) of the structural formula below and compound (ETM-2) of the structural formula below at a vapor deposition rate ratio of the compound (ETM-1):compound (ETM-2) = 50:50. The electron injection layer 8 was formed on the electron transport layer 7 by forming lithium fluoride in a film thickness of 1 nm. The cathode 9 was formed on the electron injection layer 8 by forming magnesium silver alloy in a film thickness of 12 nm. Finally, the capping layer 10 was formed by forming the compound (CPL-1) of the structural formula below in a film thickness of 60 nm. The emission characteristics of the fabricated organic EL device were measured in the atmosphere at an ordinary temperature by applying a DC voltage. The results are summarized in Table 1.

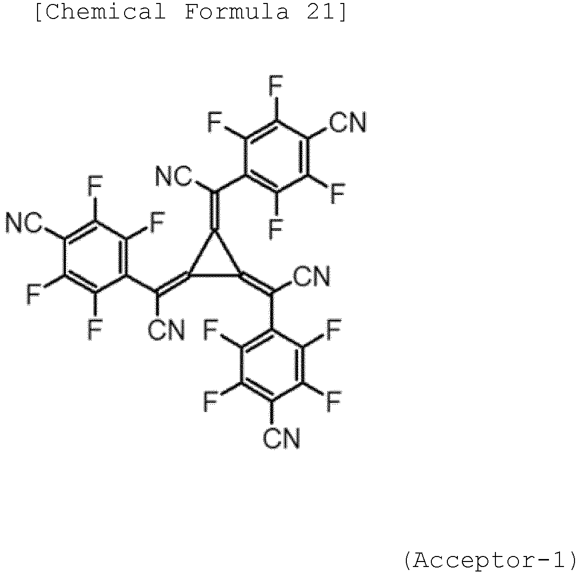

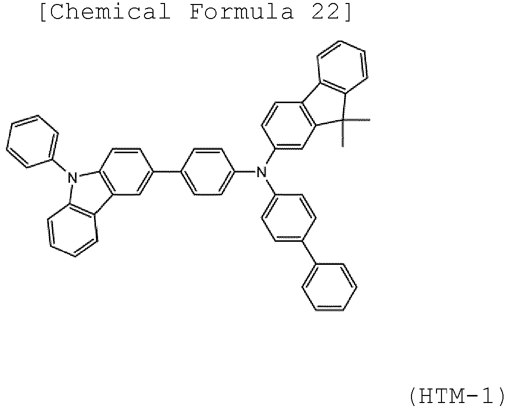

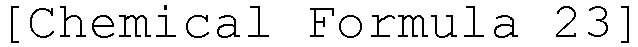

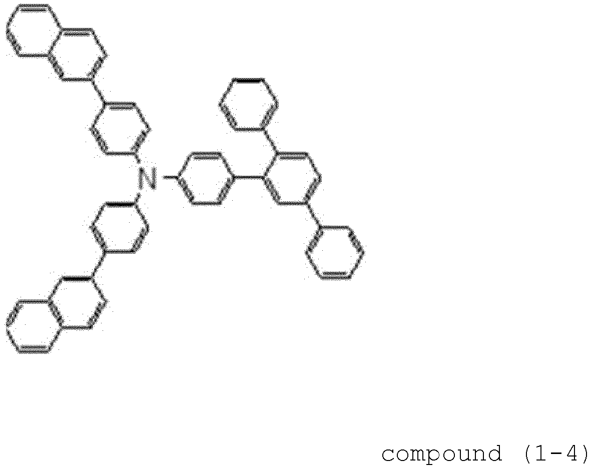

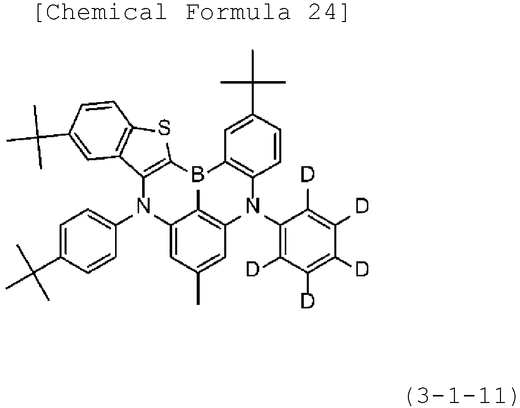

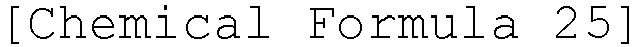

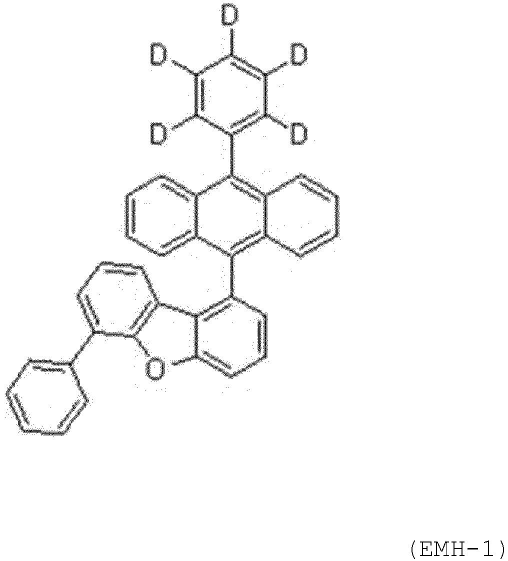

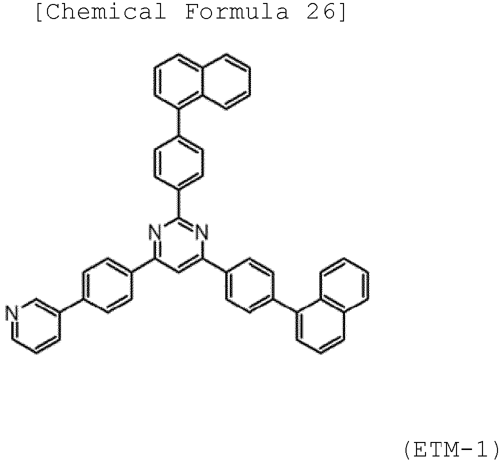

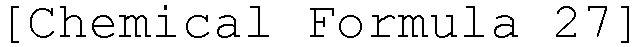

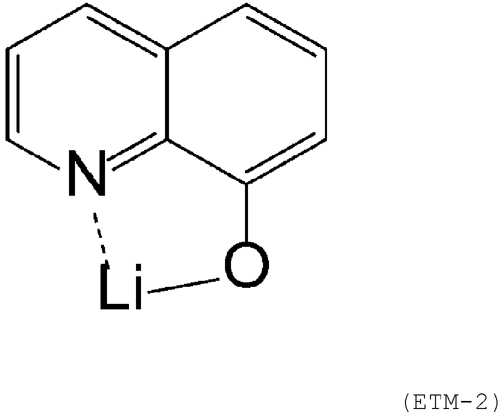

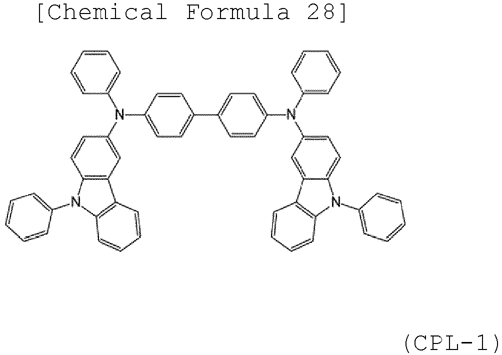

### Example 14

An organic EL device was fabricated under the same conditions used in Example 13, except that the second hole transport layer 5 was formed by forming the compound (1-58) of Example 2, instead of using the compound (1-4) of Example 1. The emission characteristics of the fabricated organic EL device were measured in the atmosphere at an ordinary temperature by applying a DC voltage. The results are summarized in Table 1.

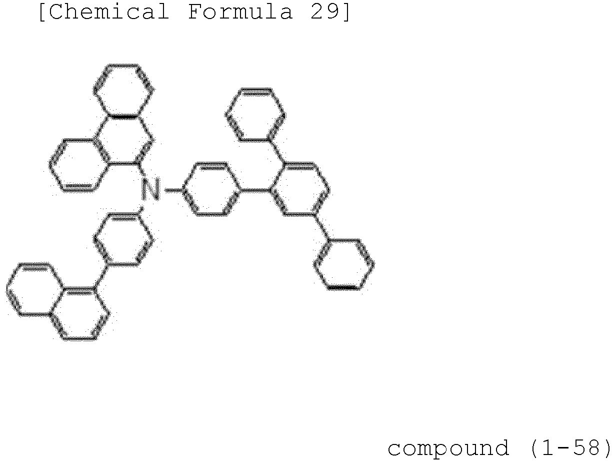

### Example 15

An organic EL device was fabricated under the same conditions used in Example 13, except that the second hole transport layer 5 was formed by forming the compound (1-59) of Example 3, instead of using the compound (1-4) of Example 1. The emission characteristics of the fabricated organic EL device were measured in the atmosphere at an ordinary temperature by applying a DC voltage. The results are summarized in Table 1.

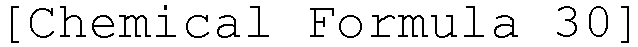

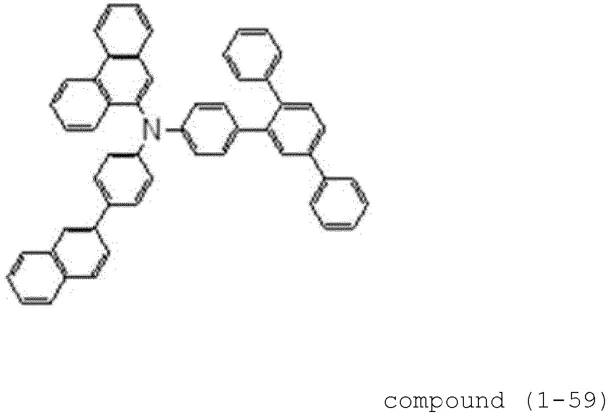

### Example 16

An organic EL device was fabricated under the same conditions used in Example 13, except that the second hole transport layer 5 was formed by forming the compound (1-69) of Example 4, instead of using the compound (1-4) of Example 1. The emission characteristics of the fabricated organic EL device were measured in the atmosphere at an ordinary temperature by applying a DC voltage. The results are summarized in Table 1.

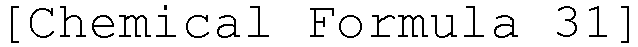

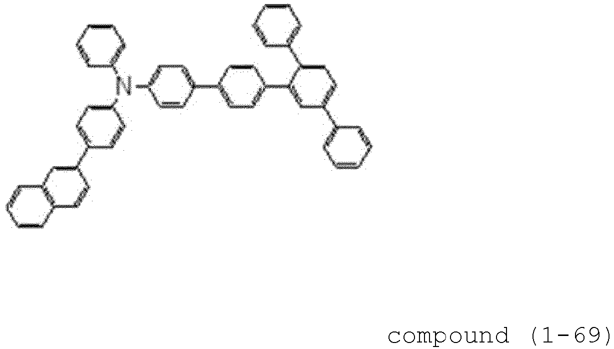

### Example 17

An organic EL device was fabricated under the same conditions used in Example 13, except that the second hole transport layer 5 was formed by forming the compound (1-83) of Example 5, instead of using the compound (1-4) of Example 1. The emission characteristics of the fabricated organic EL device were measured in the atmosphere at an ordinary temperature by applying a DC voltage. The results are summarized in Table 1.

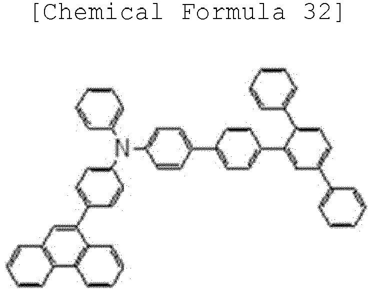

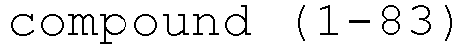

### Example 18

An organic EL device was fabricated under the same conditions used in Example 13, except that the second hole transport layer 5 was formed by forming the compound (1-108) of Example 6, instead of using the compound (1-4) of Example 1. The emission characteristics of the fabricated organic EL device were measured in the atmosphere at an ordinary temperature by applying a DC voltage. The results are summarized in Table 1.

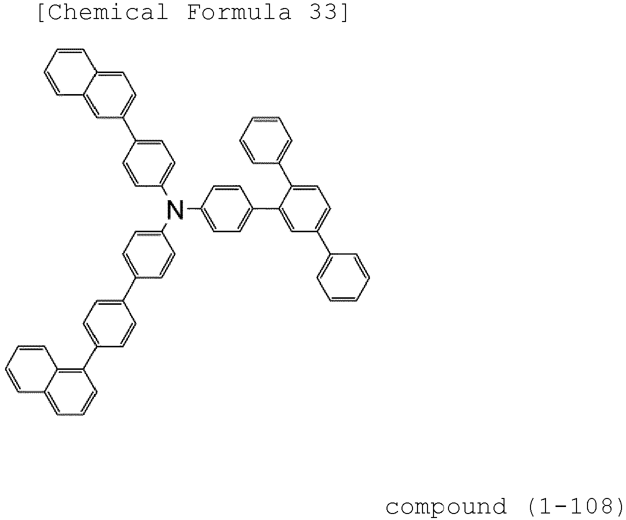

### Example 19

An organic EL device was fabricated under the same conditions used in Example 13, except that the second hole transport layer 5 was formed by forming the compound (1-112) of Example 7, instead of using the compound (1-4) of Example 1. The emission characteristics of the fabricated organic EL device were measured in the atmosphere at an ordinary temperature by applying a DC voltage. The results are summarized in Table 1.

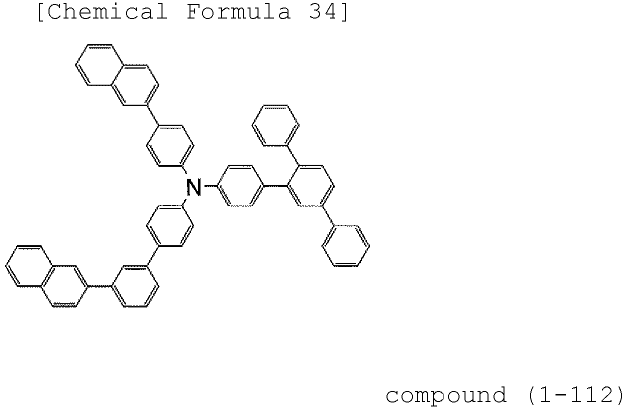

### Example 20

An organic EL device was fabricated under the same conditions used in Example 13, except that the second hole transport layer 5 was formed by forming the compound (1-145) of Example 8, instead of using the compound (1-4) of Example 1. The emission characteristics of the fabricated organic EL device were measured in the atmosphere at an ordinary temperature by applying a DC voltage. The results are summarized in Table 1.

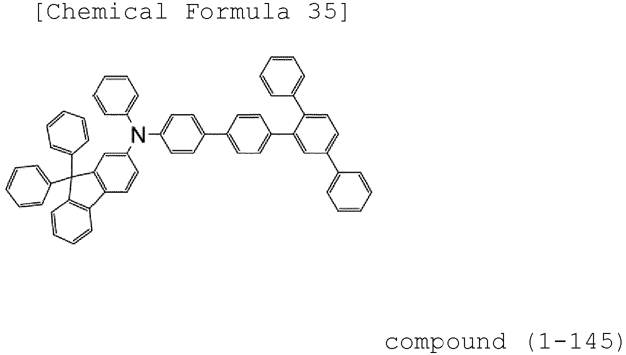

### Example 21

An organic EL device was fabricated under the same conditions used in Example 13, except that the second hole transport layer 5 was formed by forming the compound (1-174) of Example 9, instead of using the compound (1-4) of Example 1. The emission characteristics of the fabricated organic EL device were measured in the atmosphere at an ordinary temperature by applying a DC voltage. The results are summarized in Table 1.

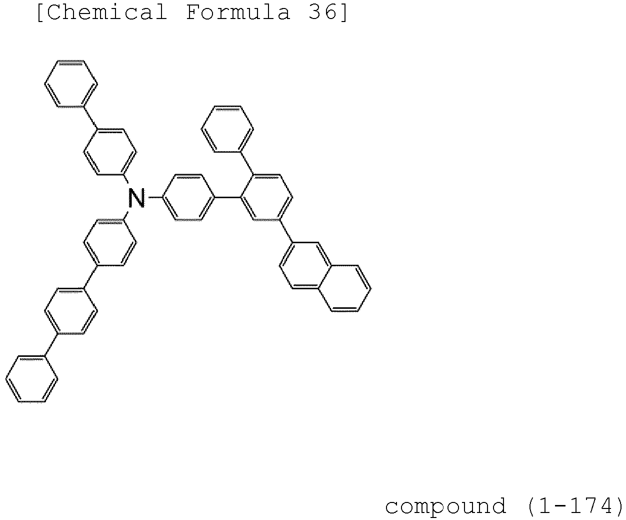

### Comparative Example 1

For comparison, an organic EL device was fabricated under the same conditions used in Example 13, except that the second hole transport layer 5 was formed by forming a compound (HTM-2) of the structural formula below, instead of using the compound (1-4) of Example 1. The emission characteristics of the fabricated organic EL device were measured in the atmosphere at an ordinary temperature by applying a DC voltage. The results are summarized in Table 1.

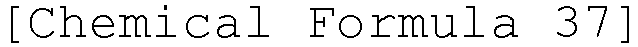

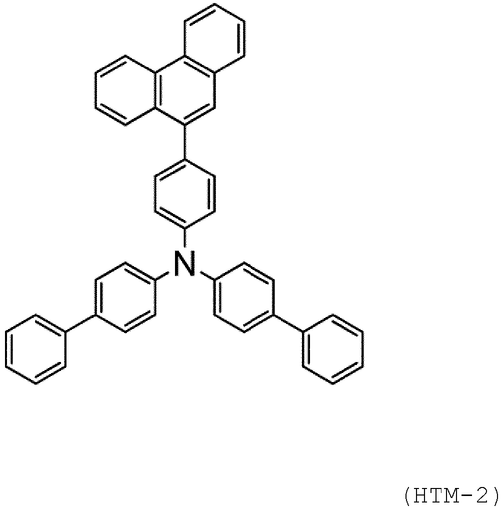

### Comparative Example 2

For comparison, an organic EL device was fabricated under the same conditions used in Example 13, except that the second hole transport layer 5 was formed by forming a compound (HTM-3) of the structural formula below, instead of using the compound (1-4) of Example 1. The emission characteristics of the fabricated organic EL device were measured in the atmosphere at an ordinary temperature by applying a DC voltage. The results are summarized in Table 1.

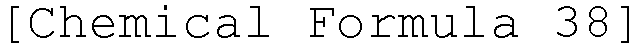

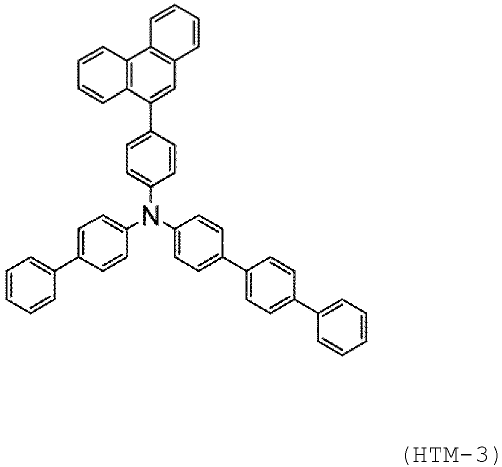

The device lifetime was measured with the organic EL devices fabricated in Examples and Comparative Examples. The results are summarized in Table 1. A device lifetime was measured as the time elapsed until the emission luminance of 2,000 cd/m² (initial luminance) at the start of emission was attenuated to 1,900 cd/m² (corresponding to attenuation to 95% when taking the initial luminance as 100%) when carrying out constant current driving.

**[Table 1]**

| | **Second hole transport layer** | **(@10mA/cm²)** | | | | **Device lifetime** |
|---|---|---|---|---|---|---|
| | | **Voltage [V]** | **Luminance [cd/m²]** | **Luminous efficiency [cd/A]** | **Power efficiency [lm/W]** | **(Attenuation to 95%)** |
| **Ex. 13** | **Compound (1-4)** | **3.41** | **957** | **9.57** | **8.83** | **505 h** |
| **Ex. 14** | **Compound (1-58)** | **3.46** | **988** | **9.88** | **8.97** | **303 h** |
| **Ex. 15** | **Compound (1-59)** | **3.44** | **968** | **9.69** | **8.85** | **391 h** |
| **Ex. 16** | **Compound (1-69)** | **3.41** | **1049** | **10.49** | **9.65** | **601 h** |
| **Ex. 17** | **Compound (1-83)** | **3.46** | **1017** | **10.16** | **9.25** | **327 h** |
| **Ex. 18** | **Compound (1-108)** | **3.43** | **1017** | **10.17** | **9.33** | **539 h** |
| **Ex. 19** | **Compound (1-112)** | **3.42** | **1034** | **10.34** | **9.45** | **645 h** |
| **Ex. 20** | **Compound (1-145)** | **3.45** | **1052** | **10.52** | **9.75** | **533 h** |
| **Ex. 21** | **Compound (1-174)** | **3.38** | **980** | **9.78** | **9.27** | **498 h** |
| **Com. Ex. 1** | **HTM-2** | **3.52** | **897** | **8.97** | **8.19** | **245 h** |
| **Com. Ex. 2** | **HTM-3** | **3.55** | **742** | **7.42** | **6.75** | **223 h** |

As shown in Table 1, the luminous efficiency upon passing a current with a current density of 10 mA/cm² was 9.57 to 10.52 cd/A for the organic EL devices in Examples 13 to 21, which was clearly higher than 7.42 to 8.97 cd/A for the organic EL devices in Comparative Examples 1 and 2. Further, the power efficiency was 8.83 to 9.75 lm/W for the organic EL devices in Examples 13 to 21, which was clearly higher than 6.75 to 8.19 lm/W for the organic EL devices in Comparative Examples 1 and 2. Table 1 also shows that the device lifetime (attenuation to 95%) was 303 to 645 hours for the organic EL devices in Examples 13 to 21, showing achievement of a significantly far longer lifetime than 223 to 245 hours for the organic EL devices in Comparative Examples 1 and 2.

As is clear from the above results, a triarylamine compound having a specific structure represented by the general formula (1) has a large mobility of holes and has an excellent electron blocking ability compared to the conventional triarylamine compound used in the devices of the comparative examples. Thus, in the organic EL device used together with the material used in the light emitting layer of the present invention, it can be seen that an organic EL device having high luminous efficiency and a long life can be realized as compared with conventional organic EL devices.

### Industrial Applicability

In the organic EL device of the present invention in which a triarylamine compound having a specific structure are used, luminous efficiency and also durability of the organic EL device can be improved compared to conventional organic EL device. Thus, for example, the development of applications in home electric appliances and illuminations can be realized.

- 1: Glass substrate
- 2: Transparent anode
- 3: Hole injection layer
- 4: First hole transport layer
- 5: Second hole transport layer
- 6: Light emitting layer
- 7: Electron transport layer
- 8: Electron injection layer
- 9: Cathode
- 10: Capping layer

## Claims

1. An organic electroluminescence device comprising at least a first hole transport layer (4), a second hole transport layer (5), a blue light emitting layer (6) and an electron transport layer (7) in this order from the anode side between anode (2) and cathode (9), wherein the second hole transport layer (5), or at least one layer of the laminated layers disposed between the first hole transport layer (4) and the electron transport layer (7) comprises a triarylamine compound represented by the following general formula (1): 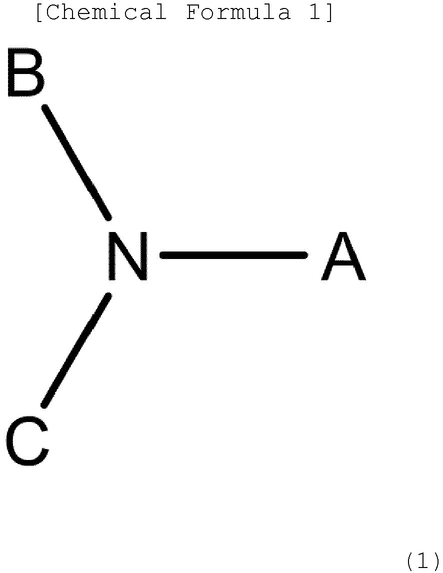
wherein A, B, and C may be the same or different, and represent a monovalent group of the general formula (2-1),
where the dashed part is the bonding site, a substituted or unsubstituted aromatic hydrocarbon group, a substituted or unsubstituted aromatic heterocyclic group, or a substituted or unsubstituted condensed polycyclic aromatic group. However, all of A, B and C shall not simultaneously be monovalent groups represented by the following general formula (2-1). 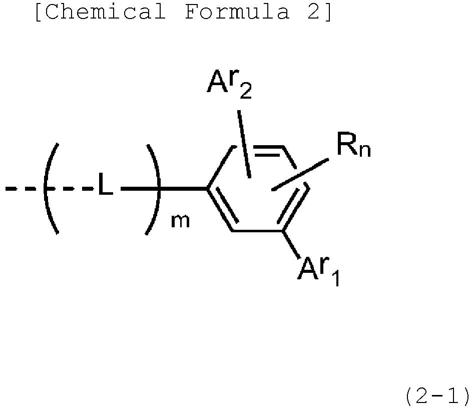
wherein the dashed part is the binding site. R represents a deuterium atom, a fluorine atom, a chlorine atom, a cyano group, a nitro group, a linear or branched alkyl group of 1 to 6 carbon atoms that may have a substituent, a cycloalkyl group of 5 to 10 carbon atoms that may have a substituent, a linear or branched alkenyl group of 2 to 6 carbon atoms that may have a substituent, a linear or branched alkyloxy group of 1 to 6 carbon atoms that may have a substituent, a cycloalkyloxy group of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or a substituted or unsubstituted aryloxy group. n is the number of R and represents an integer between 0 and 3. When n is 2 or 3, a plurality of R which bind to the same benzene ring, may be the same or different and may bind to each other via a single bond, substituted or unsubstituted methylene group, an oxygen atom, or a sulfur atom to form a ring. L represents a divalent group of a substituted or unsubstituted aromatic hydrocarbon, a divalent group of a substituted or unsubstituted aromatic heterocyclic ring, or a divalent group of substituted or unsubstituted condensed polycyclic aromatics. m represents an integer between 1 and 3. When m is 2 or 3, L may be the same or different. Ar₁ and Ar₂ may be the same or different, and represent a substituted or unsubstituted aromatic hydrocarbon group, a substituted or unsubstituted aromatic heterocyclic group, or a substituted or unsubstituted condensed polycyclic aromatic group,
wherein the blue light emitting layer (6) includes a blue light emitting dopant, and
wherein the blue light emitting dopant is a compound represented by the following general formula (3-1) or general formula (3-2), 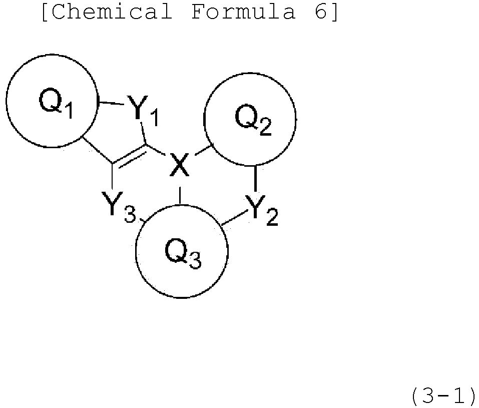 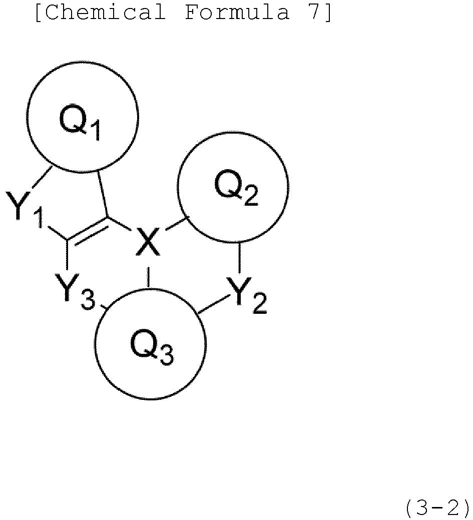
wherein, Q₁ to Q₃ may be the same or different, and represent a substituted or unsubstituted aromatic hydrocarbon, a substituted or unsubstituted condensed polycyclic aromatics, or a substituted or unsubstituted aromatic heterocyclic ring, X represents B, P, P = O, or P = S, Y₁ to Y₃ may be the same or different, and represent one of selected from N-R₄, CR₅R₆, O, S, Se and SiR₇R₈, R₄ to R₈ may be the same or different, and represent a hydrogen atom, a deuterium atom, a fluorine atom, a chlorine atom, a cyano group, a nitro group, a linear or branched alkyl group of 1 to 6 carbon atoms that may have a substituent, a cycloalkyl group of 5 to 10 carbon atoms that may have a substituent, a linear or branched alkenyl group of 2 to 6 carbon atoms that may have a substituent, a linear or branched alkyloxy group of 1 to 6 carbon atoms that may have a substituent, a cycloalkyloxy group of 5 to 10 carbon atoms that may have a substituent, a substituted or unsubstituted aromatic hydrocarbon group, a substituted or unsubstituted condensed polycyclic aromatic group, or a substituted or unsubstituted aryloxy group, R₅ and R₆, and R₇ and R₈ may bind to each other to form a ring via a single bond, substituted or unsubstituted methylene group, an oxygen atom, or a sulfur atom, when Y₁ to Y₃ are N-R₄, CR₅R₆, or SiR₇R₈, R₄ to R₈ may bind to the adjacent Q₁, Q₂, or Q₃, respectively, to form a ring via a linking group, such as substituted or unsubstituted methylene group, an oxygen atom, a sulfur atom, or a monosubstituted amino group, and
**characterised in that** the aromatic hydrocarbon, the condensed polycyclic aromatics, and the aromatic heterocyclic ring in the substituted or unsubstituted aromatic hydrocarbon, the substituted or unsubstituted condensed polycyclic aromatics, and the substituted or unsubstituted aromatic heterocyclic ring represented by Q₁ to Q₃ in the general formula (3-1) and the general formula (3-2) are selected from the group consisting of benzene, naphthalene, anthracene, fluorene, phenanthrene, pyridine, pyrimidine, triazine, pyrrole, quinoline, isoquinoline, indene, indole, indoline, carbazole, carboline, benzoxazole, benzothiazole, quinoxaline, benzimidazole, pyrazole, dibenzofuran, dibenzothiophene, naphthyridine, phenanthroline, and acridine.

2. The organic electroluminescent device according to claim 1, wherein the monovalent group represented by the general formula (2-1) is the monovalent group represented by the following general formula (2-2): 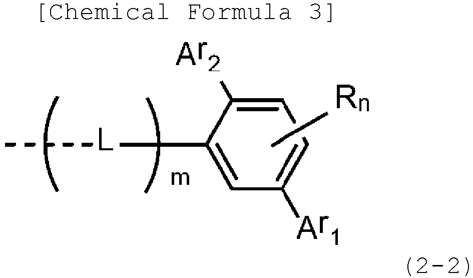 wherein the dashed part is the binding site. Ar₁, Ar₂, L, m, n and R are the same as defined by the general formula (2-1).

3. The organic electroluminescent device according to claim 1, wherein the monovalent group represented by the general formula (2-1) is the monovalent group represented by the following general formula (2-3): 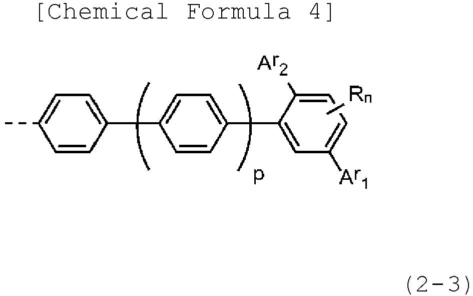 wherein the dashed part is the binding site. Ar₁, Ar₂, n and R are the same as defined by the general formula (2-1). p represents 0 or 1.

4. The organic electroluminescent device according to claim 1, wherein the monovalent group represented by the general formula (2-1) is the monovalent group represented by the following general formula (2-4): 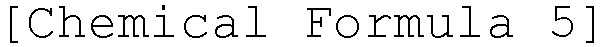 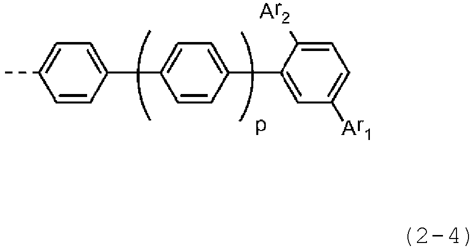 wherein the dashed part is the binding site. Ar₁, and Ar₂ are the same as defined by the general formula (2-1). p represents 0 or 1.

5. The organic electroluminescent device according to any one of claims 1 to 4, wherein the blue light emitting layer (6) includes an anthracene derivative having an anthracene skeleton in the molecule.

## Patentansprüche

1. Organisches elektrolumineszentes Element, umfassend mindestens eine erste Lochtransportschicht (4), eine zweite Lochtransportschicht (5), eine blaues Licht emittierende Schicht (6) und eine Elektronentransportschicht (7) in dieser Reihenfolge von der Anodenseite zwischen Anode (2) und Kathode (9), wobei die zweite Lochtransportschicht (5) oder mindestens eine Schicht der laminierten Schichten, die zwischen der ersten Lochtransportschicht (4) und der Elektronentransportschicht (7) angeordnet sind, eine durch die folgende allgemeine Formel (1) dargestellte Triarylaminverbindung umfasst: 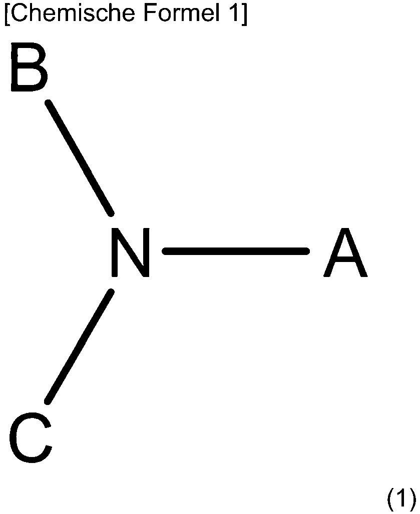
wobei A, B und C gleich oder verschieden sein können und eine einwertige Gruppe der allgemeinen Formel (2-1) darstellen, wobei der gestrichelte Teil die Bindungsstelle, eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe ist. Allerdings dürfen alle von A, B und C nicht gleichzeitig durch die folgende allgemeine Formel (2-1) dargestellte einwertige Gruppen sein. 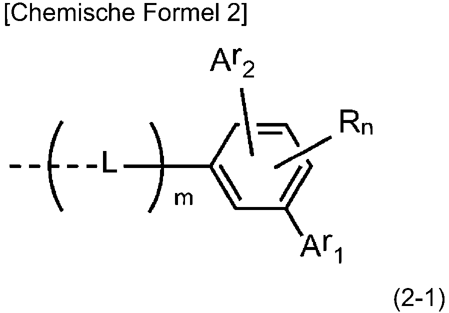
wobei der gestrichelte Teil die Bindungsstelle ist. R stellt ein Deuteriumatom, ein Fluoratom, ein Chloratom, eine Cyanogruppe, eine Nitrogruppe, eine lineare oder verzweigte Alkylgruppe aus 1 bis 6 Kohlenstoffatomen, die einen Substituenten aufweisen kann, eine Cycloalkylgruppe aus 5 bis 10 Kohlenstoffatomen, die einen Substituenten aufweisen kann, eine lineare oder verzweigte Alkenylgruppe aus 2 bis 6 Kohlenstoffatomen, die einen Substituenten aufweisen kann, eine lineare oder verzweigte Alkyloxygruppe aus 1 bis 6 Kohlenstoffatomen, die einen Substituenten aufweisen kann, eine Cycloalkyloxygruppe aus 5 bis 10 Kohlenstoffatomen, die einen Substituenten aufweisen kann, eine substituierte oder unsubstituierte aromatische Kohlenwasserstoffgruppe, eine substituierte oder unsubstituierte aromatische heterocyclische Gruppe, eine substituierte oder unsubstituierte kondensierte polycyclische aromatische Gruppe oder eine substituierte oder unsubstituierte Aryloxygruppe dar. n ist die Anzahl von R und stellt eine ganze Zahl zwischen 0 und 3 dar. Wenn n 2 oder 3 beträgt, können eine Mehrzahl von R, die an denselben Benzolring binden, gleich oder verschieden sein und aneinander über eine Einfachbindung, eine substituierte oder unsubstituierte Methylengruppe, ein Sauerstoffatom oder ein Schwefelatom binden, um einen Ring zu bilden. L stellt eine zweiwertige Gruppe eines substituierten oder unsubstituierten aromatischen Kohlenwasserstoffs, eine zweiwertige Gruppe eines substituierten oder unsubstituierten aromatischen heterocyclischen Rings oder eine zweiwertige Gruppe eines substituierten oder unsubstituierten kondensierten polycyclischen Aromaten dar. m stellt eine ganze Zahl zwischen 1 und 3 dar. Wenn m 2 oder 3 beträgt, kann L gleich oder verschieden sein. Ar₁ und Ar₂ können gleich oder verschieden sein und stellen eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe dar,
wobei die blaues Licht emittierende Schicht (6) ein blaues Licht emittierendes Dotiermittel enthält und
wobei das blaues Licht emittierende Dotiermittel eine durch die folgende allgemeine Formel (3-1) oder allgemeine Formel (3-2) dargestellte Verbindung ist, 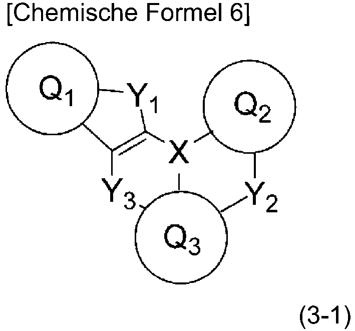 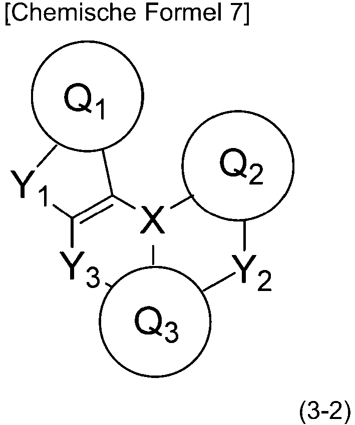
wobei Q₁ bis Q₃ gleich oder verschieden sein können und einen substituierten oder unsubstituierten aromatischen Kohlenwasserstoff, einen substituierten oder unsubstituierten kondensierten polyzyklischen Aromaten oder einen substituierten oder unsubstituierten aromatischen heterocyclischen Ring darstellen, X B, P, P = O oder P = S darstellt, Y₁ bis Y₃ gleich oder verschieden sein können und eines, ausgewählt aus N-R₄, CR₅R₆, O, S, Se und SiR₇R₈, darstellen, R₄ bis R₈ gleich oder verschieden sein können und ein Wasserstoffatom, ein Deuteriumatom, ein Fluoratom, ein Chloratom, eine Cyanogruppe, eine Nitrogruppe, eine lineare oder verzweigte Alkylgruppe aus 1 bis 6 Kohlenstoffatomen, die einen Substituenten aufweisen kann, eine Cycloalkylgruppe aus 5 bis 10 Kohlenstoffatomen, die einen Substituenten aufweisen kann, eine lineare oder verzweigte Alkenylgruppe aus 2 bis 6 Kohlenstoffatomen, die einen Substituenten aufweisen kann, eine lineare oder verzweigte Alkyloxygruppe aus 1 bis 6 Kohlenstoffatomen, die einen Substituenten aufweisen kann, eine Cycloalkyloxygruppe aus 5 bis 10 Kohlenstoffatomen, die einen Substituenten aufweisen kann, eine substituierte oder unsubstituierte aromatische Kohlenwasserstoffgruppe, eine substituierte oder unsubstituierte kondensierte polycyclische aromatische Gruppe oder eine substituierte oder unsubstituierte Aryloxygruppe darstellen, R₅ und R₆ sowie R₇ und R₈ aneinander über eine Einfachbindung, eine substituierte oder unsubstituierte Methylengruppe, ein Sauerstoffatom oder ein Schwefelatom binden können, um einen Ring zu bilden, wenn Y₁ bis Y₃ N-R₄, CR₅R₆ oder SiR₇R₈ sind, R₄ bis R₈ jeweils an das benachbarte Q₁, Q₂ oder Q₃ binden können, um über eine Verbindungsgruppe, wie beispielsweise eine substituierte oder unsubstituierte Methylengruppe, ein Sauerstoffatom, ein Schwefelatom oder eine monosubstituierte Aminogruppe, einen Ring zu bilden, und **dadurch gekennzeichnet, dass** der aromatische Kohlenwasserstoff, die kondensierten polyzyklischen Aromaten und der aromatische heterocyclische Ring in dem substituierten oder unsubstituierten aromatischen Kohlenwasserstoff, den substituierten oder unsubstituierten kondensierten polyzyklischen Aromaten und dem substituierten oder unsubstituierten aromatischen heterocyclischen Ring, die in der allgemeinen Formel (3-1) und der allgemeinen Formel (3-2) durch Q₁ bis Q₃ dargestellt sind, ausgewählt sind aus der Gruppe, bestehend aus Benzol, Naphthalin, Anthracen, Fluoren, Phenanthren, Pyridin, Pyrimidin, Triazin, Pyrrol, Chinolin, Isochinolin, Inden, Indol, Indolin, Carbazol, Carbolin, Benzoxazol, Benzothiazol, Chinoxalin, Benzimidazol, Pyrazol, Dibenzofuran, Dibenzothiophen, Naphthyridin, Phenanthrolin und Acridin.

2. Organisches elektrolumineszentes Element nach Anspruch 1, wobei die durch die allgemeine Formel (2-1) dargestellte einwertige Gruppe die durch die folgende allgemeine Formel (2-2) dargestellte einwertige Gruppe ist: 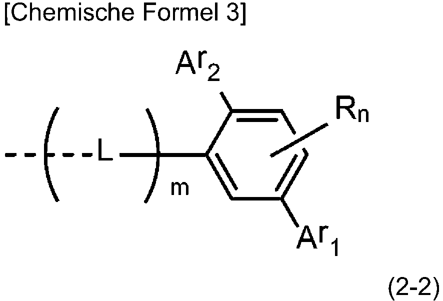 wobei der gestrichelte Teil die Bindungsstelle ist. Ar₁, Ar₂, L, m, n und R sind dieselben wie durch die allgemeine Formel (2-1) definiert.

3. Organisches elektrolumineszentes Element nach Anspruch 1, wobei die durch die allgemeine Formel (2-1) dargestellte einwertige Gruppe die durch die folgende allgemeine Formel (2-3) dargestellte einwertige Gruppe ist: 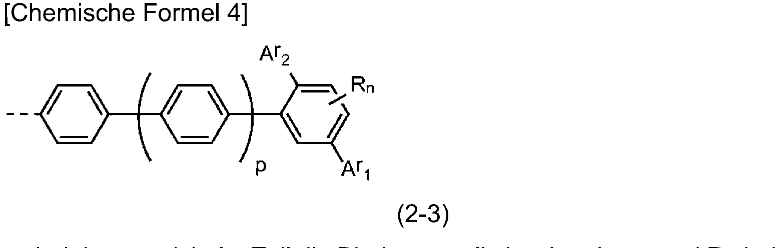 wobei der gestrichelte Teil die Bindungsstelle ist. Ar₁, Ar₂, n und R sind dieselben wie durch die allgemeine Formel (2-1) definiert. p stellt 0 oder 1 dar.

4. Organisches elektrolumineszentes Element nach Anspruch 1, wobei die durch die allgemeine Formel (2-1) dargestellte einwertige Gruppe die durch die folgende allgemeine Formel (2-4) dargestellte einwertige Gruppe ist: 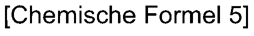 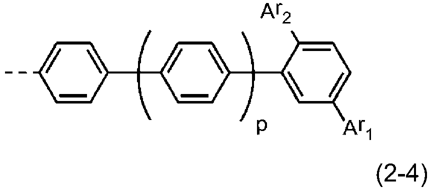 wobei der gestrichelte Teil die Bindungsstelle ist. Ar₁ und Ar₂ sind dieselben wie durch die allgemeine Formel (2-1) definiert. p stellt 0 oder 1 dar.

5. Organisches elektrolumineszentes Element nach einem der Ansprüche 1 bis 4, wobei die blaues Licht emittierende Schicht (6) ein Anthracenderivat mit einem Anthracengerüst in dem Molekül beinhaltet.

## Revendications

1. Dispositif électroluminescent organique comprenant au moins une première couche de transport de trous (4), une seconde couche de transport de trous (5), une couche d'émission de lumière bleue (6) et une couche de transport d'électrons (7) dans cet ordre à partir du côté d'anode entre l'anode (2) et la cathode (9), dans lequel la seconde couche de transport de trous (5), ou au moins une couche des couches stratifiées disposées entre la première couche de transport de trous (4) et la couche de transport d'électrons (7), comprend un composé de triarylamine représenté par la formule générale suivante (1) : 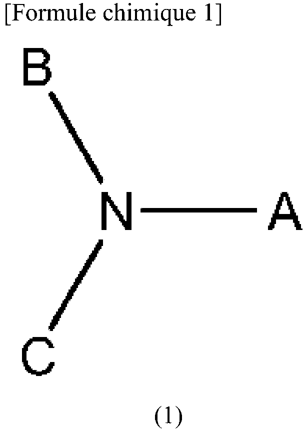
dans laquelle A, B et C peuvent être identiques ou différents, et représentent un groupe monovalent de la formule générale (2-1), où la partie en pointillés est le site de liaison, un groupe hydrocarbure aromatique substitué ou non substitué, un groupe hétérocyclique aromatique substitué ou non substitué ou un groupe aromatique polycyclique condensé substitué ou non substitué. Toutefois, l'ensemble de A, B et C ne doit pas simultanément être des groupes monovalents représentés par la formule générale suivante (2-1). 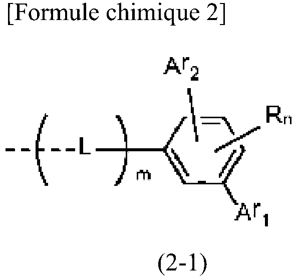
dans laquelle la partie en pointillés est le site de liaison. R représente un atome de deutérium, un atome de fluor, un atome de chlore, un groupe cyano, un groupe nitro, un groupe alkyle linéaire ou ramifié de 1 à 6 atomes de carbone qui peut avoir un substituant, un groupe cycloalkyle de 5 à 10 atomes de carbone qui peut avoir un substituant, un groupe alkényle linéaire ou ramifié de 2 à 6 atomes de carbone qui peut avoir un substituant, un groupe alkyloxy linéaire ou ramifié de 1 à 6 atomes de carbone qui peut avoir un substituant, un groupe cycloalkyloxy de 5 à 10 atomes de carbone qui peut avoir un substituant, un groupe hydrocarbure aromatique substitué ou non substitué, un groupe hétérocyclique aromatique substitué ou non substitué, un groupe aromatique polycyclique condensé substitué ou non substitué, ou un groupe aryloxy substitué ou non substitué. n est le nombre de R et représente un entier entre 0 et 3. Lorsque n est 2 ou 3, une pluralité de R qui se lient au même anneau benzénique, peuvent être identiques ou différents et peuvent se lier l'un à l'autre par le biais d'une liaison simple, d'un groupe méthylène substitué ou non substitué, d'un atome d'oxygène ou d'un atome de soufre pour former un anneau. L représente un groupe divalent d'un hydrocarbure aromatique substitué ou non substitué, un groupe divalent d'un anneau hétérocyclique aromatique substitué ou non substitué ou un groupe divalent d'un composé aromatique polycyclique condensé substitué ou non substitué. m représente un entier entre 1 et 3. Lorsque m est 2 ou 3, L peut être identique ou différent. Ar₁ et Ar₂ peuvent être identiques ou différents, et représentent un groupe hydrocarbure aromatique substitué ou non substitué, un groupe hétérocyclique aromatique substitué ou non substitué ou un groupe aromatique polycyclique condensé substitué ou non substitué,
dans lequel la couche d'émission de lumière bleue (6) inclut un dopant d'émission de lumière bleue, et
dans lequel le dopant d'émission de lumière bleue est un composé représenté par la formule générale (3-1) ou formule générale (3-2) suivante, 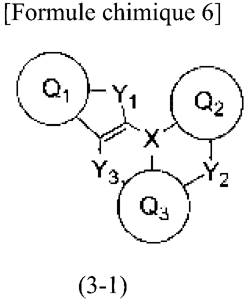 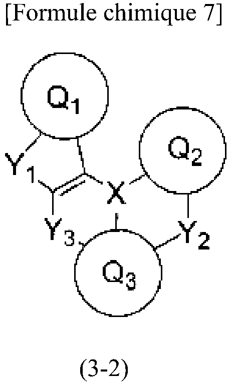
dans lesquelles Q₁ à Q₃ peuvent être identiques ou différents, et représentent un hydrocarbure aromatique substitué ou non substitué, un composé aromatique polycyclique condensé substitué ou non substitué, ou un anneau hétérocyclique aromatique substitué ou non substitué, X représente B, P, P = O, ou P = S, Y₁ à Y₃ peuvent être identiques ou différents, et représentent un composé parmi des composés sélectionnés parmi N-R₄, CR₅R₆, O, S, Se et SiR₇R₈, R₄ à R₈ peuvent être identiques ou différents, et représentent un atome d'hydrogène, un atome de deutérium, un atome de fluor, un atome de chlore, un groupe cyano, un groupe nitro, un groupe alkyle linéaire ou ramifié de 1 à 6 atomes de carbone qui peut avoir un substituant, un groupe cycloalkyle de 5 à 10 atomes de carbone qui peut avoir un substituant, un groupe alkényle linéaire ou ramifié de 2 à 6 atomes de carbone qui peut avoir un substituant, un groupe alkyloxy linéaire ou ramifié de 1 à 6 atomes de carbone qui peut avoir un substituant, un groupe cycloalkyloxy de 5 à 10 atomes de carbone qui peut avoir un substituant, un groupe hydrocarbure aromatique substitué ou non substitué, un groupe aromatique polycyclique condensé substitué ou non substitué, ou un groupe aryloxy substitué ou non substitué, R₅ et R₆, et R₇ et R₈ peuvent se lier l'un à l'autre pour former un anneau par le biais d'une liaison simple, d'un groupe méthylène substitué ou non substitué, d'un atome d'oxygène ou d'un atome de soufre, lorsque Y₁ à Y₃ sont N-R₄, CR₅R₆ ou SiR₇R₈, R₄ à R₈ peuvent se lier au Q₁, Q₂ ou Q₃ adjacent, respectivement, pour former un anneau par le biais d'un groupe de liaison, tel qu'un groupe méthylène substitué ou non substitué, un atome d'oxygène, un atome de soufre ou un groupe amino monosubstitué, et **caractérisé en ce que**
l'hydrocarbure aromatique, le composé aromatique polycyclique condensé et l'anneau hétérocyclique aromatique dans l'hydrocarbure aromatique substitué ou non substitué, le composé aromatique polycyclique condensé substitué ou non substitué et l'anneau hétérocyclique aromatique substitué ou non substitué représentés par Q₁ à Q₃ dans la formule générale (3-1) et la formule générale (3-2) sont sélectionnés parmi le groupe constitué du benzène, naphtalène, anthracène, fluorène, phénanthrène, de la pyridine, pyrimidine, triazine, du pyrrole, de la quinoline, de l'isoquinoline, de l'indène, de l'indole, du carbonazole, de la carboline, du benzoxazole, benzothiazole, de la quinoxaline, du benzimidazole, pyrazole, dibenzofurane, dibenzothiophène, de la naphtyridine, phénanthroline et de l'acridine.

2. Dispositif électroluminescent organique selon la revendication 1, dans lequel le groupe monovalent représenté par la formule générale (2-1) est le groupe monovalent représenté par la formule générale suivante (2-2) : 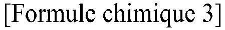 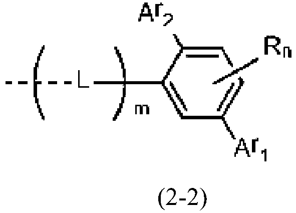 dans laquelle la partie en pointillés est le site de liaison. Ar₁, Ar₂, L, m, n et R sont identiques à la définition par la formule générale (2-1).

3. Dispositif électroluminescent organique selon la revendication 1, dans lequel le groupe monovalent représenté par la formule générale (2-1) est le groupe monovalent représenté par la formule générale suivante (2-3) : 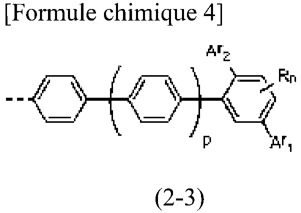 dans laquelle la partie en pointillés est le site de liaison. Ar₁, Ar₂, n et R sont identiques à la définition par la formule générale (2-1). p représente 0 ou 1.

4. Dispositif électroluminescent organique selon la revendication 1, dans lequel le groupe monovalent représenté par la formule générale (2-1) est le groupe monovalent représenté par la formule générale suivante (2-4) : 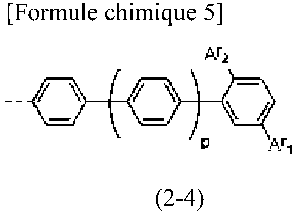 dans laquelle la partie en pointillés est le site de liaison. Ar₁ et Ar₂ sont identiques à la définition par la formule générale (2-1). p représente 0 ou 1.

5. Dispositif électroluminescent organique selon l'une quelconque des revendications 1 à 4, dans lequel la couche d'émission de lumière bleue (6) inclut un dérivé d'anthracène ayant un squelette d'anthracène dans la molécule.
